# EUROPEAN PATENT APPLICATION

(11) **EP 4 844 407 A1**
(43) Date of publication of application: **25.11.2026**
(21) Application number: 25177398.2
(22) Date of filing: 19.05.2025
(51) Int. Cl.: G01N 33/53, G01N 33/68

(54) **METHOD OF TREATING A SAMPLE**

(71) Applicant: MinervaX ApS, 2000 Frederiksberg (DK)
(72) Inventor: BAHNEN, Wael, 226 52 LUND (SE); GONZALES-MIRO, Majela, 222 21 LUND (SE); JOHANSSON LINDBOM, Bengt, 224 57 LUND (SE); ALAMIRI, Feiruz, 215 79 MALMÖ (SE)
(74) Representative: Öberg & Sjöberg AB

(57) **Abstract**

The technology proposed herein relates to a method of treating a sample, wherein the sample comprises blood, serum, and/or plasma from a human infant or from a human umbilical cord, wherein the method comprises removing, inactivating, and/or cleaving human IgM in the sample. A method of obtaining a measurement of immunological activity of a sample, a method of obtaining a surrogate endpoint, a surrogate efficacy test method, a use of an enzyme, and a kit, are also disclosed.

## Description

### FIELD OF INVENTION

The technology proposed herein relates to the fields of microbiology and vaccine technology and concerns a method of treating a sample. More particularly, the technology proposed herein relates to a method of treating a sample comprising removal, inactivation and/or cleaving of human igM in the sample.

### BACKGROUND OF THE INVENTION

Group B Streptococcus (*Streptococcus agalactiae)* (GBS) is the major cause of invasive bacterial infections, including meningitis, in the neonatal period. In the United States alone, there are now about 5000 cases per year of invasive disease caused by this bacterium. These infections have an overall mortality of about 10%, and many of the infants that survive have permanent neurological sequelae. In view of this, a large effort has been made to find methods of prevention and treatment and to analyze the mechanisms by which GBS cause infections.

About 20 % of all women are vaginal carriers of GBS, and vertical transmission from the maternal genital tract is probably the most common source of infection in neonatal disease caused by this bacterium. However, only about 1 % of the infants that are colonized by the GBS at birth are afflicted by serious infection. Other factors than exposure to the bacterium during birth must therefore contribute to the development of neonatal disease. GBS can also cause mastitis in cows, a bovine disease that is of considerable economic importance.

Development of a vaccine against GBS infections is therefore of interest. An integral part of vaccine development is the testing of vaccine efficacy. However, as the incidence of GBS disease is low, it is burdensome to perform a study whereby a sufficient large number of test subjects are vaccinated with a vaccine and a clinical endpoint, i.e. whether or not each vaccinated subject becomes seriously infected or not, is determined.

For such diseases, as well as for other diseases having undesired clinical endpoints, e.g. death, surrogate endpoints may be used as an alternative. Thus, instead of determining whether a vaccinated subject contracts infection and disease or not, the surrogate endpoint, or surrogate marker, may be determined instead. As an example, a biomarker such as the titer of antibodies against an antigen of a vaccine can be determined and correlated with titers of antibodies in subjects who were exposed to the disease naturally, i.e. without intervention, and who had not received the vaccine. In such a way a titer of antibodies in a vaccinated subject, that corresponds to or surpasses the titer of antibodies in a group of subjects that were exposed to a microbe such as a bacteria naturally but did not contract the disease, can be used to indicate that the vaccine has a clinical effect of protecting against the disease. In other words, a titer of antibodies higher than some set level, based on comparing antibody levels of subjects that did or did not contract infection and disease, is used as a surrogate endpoint instead of the clinical endpoint of contraction of disease. In particular, such surrogate endpoint thresholds may be based on a functional killing titer determined in an opsonophagocytic killing activity assay, OPkA, assay.

In view of the low incidence of serious GBS infection in infants, and in view of the undesirability of clinical trials being performed on infants, development of GBS vaccines would be greatly facilitated by the availability of a surrogate endpoints, and thereby of a surrogate efficacy test, for evaluating vaccines against GBS.

Defining, or designing, a surrogate endpoint and a surrogate efficacy test can however be difficult if it is unknown which biomarker or biomarkers correlate with the clinical endpoint. In particular, if samples from test subjects in a general population are to be evaluated to determine a surrogate endpoint, the samples may contain many different compounds that could be the basis for a certain test subject's immunity towards the disease or microbe concerned. Expressed differently, a sample's content of one biomarker may not provide a dependable answer to why the test subject associated with the sample was infected or were immune. In continuation, a surrogate endpoint and surrogate efficacy test established upon such a biomarker cannot be relied upon for determining the efficacy of a vaccine on the basis of the level of biomarker obtained by vaccination of other test subjects.

Accordingly, it is a primary objective of the technology proposed herein to provide a method of treating a sample, e.g. so that a reliable and predictable surrogate marker can be identified.

It is a second objective of the technology proposed herein to provide a method of obtaining a measurement of immunological activity of a sample.

It is a third objective of the technology proposed herein to provide a method of obtaining a surrogate endpoint.

It is a fourth objective of the technology proposed herein to provide a surrogate efficacy test method.

It is a fifth objective of the technology proposed herein to provide a use of an enzyme.

It is a sixth objective of the technology proposed herein to provide a kit.

### SUMMARY

A first aspect of the technology proposed herein relates to a method of treating a sample, wherein the sample comprises blood, serum, and/or plasma from a human infant or from a human umbilical cord, wherein the method comprises:
i. removing, inactivating, and/or cleaving human IgM in the sample.

The proposed technology is accordingly based on the discovery that, as shown in example 1, IgM could be present in serum samples from cord and infant blood while not contributing to protection against GBS in the newborn baby. First, such IgM in these samples could represent polyreactive innate IgM, produced already during fetal life, enhancing bacterial killing in the assay but lacking similar protective effect *in vivo.* Second, IgM could have been produced by the infants in response to the infection, but due to the time delay between onset of infection and blood sampling, reached levels sufficiently high to mediate bacterial killing first at the time blood was collected (i.e. too late to mediate protection *in vivo*)*.* Third, IgM reactive to GBS can be very high in the mother, and although IgM is not placentally transferred to the baby, small amounts of maternal blood could contaminate collected cord blood samples due to mechanical disruption of the placental tissue during delivery.

During initial assessments, it was unexpected that that the presence of such IgM in the cord and infant serum samples would skew OPkA titer results to the extent that no statistically significant difference could be achieved between infants afflicted by serious GBS infection (cases) and infants that remained healthy despite being exposed to GBS bacteria e.g. during birth (controls). By removing IgM from the samples, a statistically significant difference was however detectable between the two groups. This significant difference in OPkA titer between cases and controls, observed firstly after elimination of the IgM, could thus be used to establish an OPkA threshold as a surrogate endpoint marker for vaccine efficacy in clinical vaccine studies. Expressed differently, a vaccine that can induce an immune response sufficiently high to reach such OPkA threshold in the cord serum samples could be considered to provide efficient protection against neonatal GBS infection. This would allow for an evaluation of vaccine efficacy without having to wait for and monitor whether the incidence of natural neonatal infections differ between very large cohorts of vaccinated and placebo mothers.

A second aspect of the proposed technology relates to a method of obtaining a measurement of immunological activity of a sample, comprising the steps of:
i. performing the method according to the first aspect of the proposed technology, and
ii. obtaining a measurement of immunological activity of the sample against microbes.

A third aspect of the proposed technology relates to a method of obtaining a surrogate endpoint, comprising the steps of:
i. performing the method according to the second aspect of the proposed technology to obtain a measurement of immunological activity against a microbe for at least one sample associated with at least one of:
   a. a first group of infants not afflicted by disease and/or infection after having been exposed to a microbe and
   b. a second group of infants afflicted by disease and/or infection after being exposed to a microbe, and
ii. determining at least one surrogate endpoint based on the measurement.

A fourth aspect of the proposed technology relates to a surrogate efficacy test method for testing efficacy of a vaccine comprising the steps of:
i. providing at least one surrogate endpoint by performing the method according to the third aspect of the proposed technology,
ii. vaccinating at least one human using a vaccine against the microbe,
iii. performing the method according to the second aspect of the proposed technology on a sample from the at least one human, and
iv. comparing the measurement obtained with the at least one surrogate endpoint.

A fifth aspect of the proposed technology relates to a use of an enzyme as defined herein to inactivate and/or cleave human IgM in a sample comprising blood, serum, and/or plasma from a human infant or from a human umbilical cord.

A sixth aspect of the proposed technology relates to a kit comprising:
- an enzyme as defined herein, and
- a further enzyme as defined herein.

Other features and advantages of the technology proposed herein will be apparent from the following detailed description, figure, examples, and from the claims.

### BRIEF DESCRIPTION OF THE FIGURES

- Fig. 1: shows results of an OPkA performed on samples from Cases and Controls with or without cleavage of IgM in the samples.
- Fig. 2: shows results of an OPkA performed on blood samples from mothers pre-vaccination (Visit 1) and post-vaccination at delivery (Visit 5) with corresponding samples of cord blood from the infant (Visit 5) following cleavage of IgM or IgG.

### DETAILED DESCRIPTION

As stated above, a first aspect of the technology proposed herein relates to a method of treating a sample, wherein the sample comprises blood, serum, and/or plasma from a human infant or from a human umbilical cord, wherein the method comprises:
i. removing, inactivating, and/or cleaving human IgM in the sample.

The method according to the first aspect of the proposed technology, by removing, inactivating and/or cleaving human IgM in the sample, thus provides for using the sample to obtain a measurement of immunological activity of the sample, to obtain a surrogate endpoint, and to obtain a surrogate efficacy test method. Specifically, removing, inactivating and/or cleaving human IgM in the sample provides for obtaining a measurement of immunological activity of the sample which, especially for samples from human cord and infant blood, may be more representative and relevant for assessing a protective effect *in vivo.*

The method of treating a sample may alternatively be considered a method for pretreating a sample or a method for conditioning a sample. The method may treat a sample for a subsequent analysis, in particular a subsequent analysis involving determining microbial viability.

The method may be considered a method for treating a sample for obtaining one or more of a measurement of immunological activity of the sample, a surrogate endpoint, and a surrogate efficacy test method. In particular, the method may be considered a method for treating a sample for obtaining one or more of a measurement of immunological activity of the sample against GBS, a surrogate endpoint for a GBS surrogate efficacy test method, and a surrogate efficacy test method for testing efficacy of a vaccine against GBS. The GBS, i.e. the GBS bacteria or GBS strain, may be further defined as described below e.g. with reference to its expression of surface proteins and/or type of capsular polysaccharide.

Preferably the sample is a serum sample. Serum is generally the clear, yellowish fluid remaining after clotting factors (such as fibrinogen and prothrombin) have been removed from blood plasma by clot formation. Hemolysis may however give a reddish color to serum.

It is specified that the sample comprises blood, serum, and/or plasma from a human infant or from a human umbilical cord. Expressed differently, the sample has been obtained from a human infant or from a human umbilical cord. Samples comprising blood, serum, and/or plasma from human umbilical cords are routinely obtained for various uses within healthcare.

The infant is preferably a neonate. Expressed differently, the infant is preferably 0-3 months old, i.e. 0-3 months have passed since the infant was born.

The sample may be obtained by way of, for example, a standard blood test. This preferably does not involve treatment of the human or animal body by therapy or surgery. In particular, blood tests and/or blood samples are obtained routinely for various uses within healthcare, e.g. during diagnostic or therapeutic procedures, or during clinical trials, and such samples, stored in e.g. a biobank or otherwise, may be used to carry out the methods according to the various aspects of the proposed technology.

The sample is preferably obtained from at least one of:
a. a human infant, or a human umbilical cord associated therewith, not afflicted by disease and/or infection after having been exposed to a microbe and
b. a human infant, or a human umbilical cord associated therewith, afflicted by disease and/or infection after being exposed to a microbe.

A human umbilical cord associated with an infant is to be understood as referring to a human umbilical cord that has been connected to the infant.

In each case the human infant has been exposed to the microbe spontaneously e.g. by the microbe being present in the environment of the human infant. Such exposure may be from the mother of the human infant, i.e. the mother being a carrier of the microbe, or from the microbe being present in hospital or care institution in which the infant is born and/or treated. Accordingly, the methods according to the various aspects of the proposed technology do not comprise intentionally exposing a human infant to a microbe.

Alternatively, the sample is obtained from a human infant, or a human umbilical cord associated therewith, born from a mother having been vaccinated against a microbe, the microbe preferably being GBS. In each case, the step (i) of removing, inactivating and/or cleaving human IgM in the sample allows a more correct measurement of the immunological activity of the sample.

Immunoglobulin M (IgM) plays an important role in maintaining an intact primary defence, wherein IgM can neutralize microbes by inhibiting the binding between respiratory microorganism and their cognate cellular receptors. Further, IgM plays a key role in the opsonization of pathogens and activation of complement.

The human IgM is preferably directed against, i.e. binding to, GBS bacteria. In particular the human IgM is directed against, i.e. binds to, GBS surface proteins such as Rib, AlpC, Alp1, Alp2, Alp3, more preferably Rib, Alp2 or Alp3. More particularly the human IgM is directed against, i.e. binds to, the N-terminal of Rib (SEQ ID NO: 33), Alp2 (SEQ ID NO: 36) or Alp3 (SEQ ID NO: 36)

The human IgM to be removed may be alternatively or additionally be selected from the group consisting of polyreactive innate IgM or germline encoded natural IgM, IgM produced by the infant at onset of a GBS infection, and IgM originating from the mother of the infant and contaminating the sample.

It is specified that the method comprises removing, inactivating, and/or cleaving human IgM in the sample. Expressed differently, the method comprises removing, inactivating and/or cleaving at least part of human IgM in the sample. Preferably at least 50%, such as at least 80%, more preferably at least 90%, most preferably at least 95% of the human IgM in the sample is removed, inactivated and/or cleaved.

The step (i) of removing, inactivating, and/or cleaving human IgM in the sample is performed for a time sufficient to remove, inactivate and/or cleave at least a part of human IgM in the sample.

Human IgM in the sample may be removed. Accordingly, human IgM in the sample can be separated from the sample. Human IgM in the sample may be removed using, for example, a column with a matrix or beads carrying antibodies against Human IgM. The sample may thus be passed through the column whereby human IgM in the sample is retained by the matrix or beads.

Human IgM in the sample may be inactivated. Inactivated encompasses any treatment that results in inactive human igM, i.e. IgM that is no longer is capable of fulfilling the functions of human IgM such as in particular binding to a microbe and binding complement component C1 to lead to opsonization of antigens and cytolysis.

Human IgM in the sample may, for example, be inactivated by aggregation or cleaving. Aggregation may be performed by using bispecific antibodies to cause human IgM to aggregate to each other.

Human IgM in the sample is preferably cleaved or hydrolyzed. Accordingly human IgM in the sample is preferably cleaved into smaller inactive fragments.

Preferably step (i) is performed by treating the sample with an enzyme to cleave human IgM in the sample. Cleaving IgM is a preferred method of removing and/or inactivating IgM in the sample. Using an enzyme is a simple and effective way of cleaving human IgM in the sample.

Enzymes isolated from the *Lachnoanaerobaculum* genus of commensal human bacteria are able to cleave IgM with high specificity. As such, the enzymes allow for the specific and effective targeting of IgM, including human IgM.

Enzymes having IgM specific endoprotease activity may comprise or consist of a sequence derived from *Lachnoanaerobaculum umeaense.* Exemplary *Lachnoanaerobaculum umeaense* polypeptide sequences are given by SEQ ID Nos 1-7 in table 1 further below.

Alternatively, enzymes having IgM specific endoprotease activity may comprise or consist of a sequence derived from *Lachnoanaerobaculum gingivalis.* Exemplary *Lachnoanaerobaculum gingivalis* polypeptide sequences are given by SEQ ID Nos 8-11 in table 1 further below. Exemplary polynucleotide sequences that encode polypeptides having IgM specific endoprotease activity are also set out in Table 1 further below. WO2024246348A1 discloses enzymes cleaving human IgM.

Preferably the enzyme comprises or consists of a polypeptide having at least 80%, preferably at least 90% sequence identity to SEQ ID NO: 1 or SEQ ID NO: 8, or alternatively at least 80%, preferably at least 90%, sequence identity to SEQ ID NO: 2 or SEQ ID NO: 9.
Such enzymes are highly effective in cleaving human IgM.

The enzyme may further comprise or consist of a polypeptide having at least 95%, such as at least 96%, at least 97%, at least 98%, at least 99%, or 100% sequence identity to SEQ ID NO: 1 or SEQ ID NO: 8, or alternatively at least 95%, such as at least 96%, at least 97%, at least 98%, at least 99%, or 100% sequence identity to SEQ ID NO: 2 or SEQ ID NO: 9.

Preferably the polypeptide has an additional methionine at the N terminus; and/or a His tag at the C terminus or at the N-terminus.

Preferably the polypeptide comprises or consists of a sequence selected from the group consisting of SEQ ID NOs: 3, 4, 5, 6, 7, 10, and 11, wherein the polypeptide more preferably comprises or consists of SEQ ID NO: 7.

Further enzymes having IgM specific endoprotease activity may be isolated from any of *Lachnoanaerobaculum sp. ICM7, Mediterraneibacter massiliensis, Lachnospiraceae bacterium,* and *Oribacterium Sinus,* as discussed in Windgassen T et al, Identification of bacterial protease domains that cleave human IgM. Enzyme Microb Technol. 2024 Feb;173:110366. doi: 10.1016/j.enzmictec.2023.110366. Epub 2023 Nov 25. PMID: 38061198. Specifically, the enzymes may be selected among the following Accension IDs: EJP19400.1 (*Lachnoanaerobaculum sp. ICM7,* 629 AA, SEQ ID NO: 23 in table 1), WP_162858205.1 (*Mediterraneibacter massiliensis,* 613 AA, SEQ ID NO: 24 in table 1), RKW40033.1 (*Lachnospiraceae bacterium,* 740 AA, SEQ ID NO: 25 in table 1), and WP_007157024.1 (*Oribacterium sinus,* 687 AA, SEQ ID NO: 26 in table 1).

Thus, the enzyme may alternatively comprise or consist of a polypeptide having at least 95%, such as at least 96%, at least 97%, at least 98%, at least 99%, or 100% sequence identity to any of the SEQ ID Nos: 23-26.

Preferably the sample is treated with the enzyme for at least 30 minutes, such as at least 1 hour, such as at least 2 hours, and up to 30 hours, such as up to 24 hours.

The concentration of the enzyme may be 1ug/mL.

Preferably the method further comprises the step of:
ii. treating the sample with a further enzyme to inactivate an antimicrobial substance in the sample, wherein the further enzyme preferably is a beta-lactamase, preferably a carbapenemase, more preferably a class B carbapenemase.

This is advantageous in that it prevents any remaining antimicrobial substances in the sample from skewing the results of any subsequent analysis of the sample utilizing microbial viability. Where for example an OPkA is performed on the sample, any remaining antimicrobial substances in the sample could lead to an increased killing of the test bacteria which was not due to opsonization, i.e. not due to the immunological activity of the sample. Step (ii) thus further improves the method according to the first aspect of the proposed technology.

Step (ii) may be performed before, together with, or after step (i).

The sample may comprise the antimicrobial substance due to the human infant, or its mother, having been treated with the antimicrobial substance. Antimicrobial substances are often used in healthcare, and their use may in some cases not have been documented. Alternatively, the information that an antimicrobial substance may be present in a sample may not be available when treating a sample as per the methods according to the various aspects of the proposed technology. Accordingly, including step (ii) in the method thus increases the number of samples that can be used and obviates the need to analyze a sample for remaining antimicrobial substances.

An analysis of the sample utilizing microbial viability is to be understood as encompassing an analysis in which the viability of microbes is determined, and in particular an analysis in which the viability of microbes is used as an indication for determining a property of the sample. Viability of a microbe may encompass any of whether a microbe or population of microbes survives, multiplies, goes dormant, or dies.

The microbes are preferably bacteria, more preferably GBS bacteria.

Treating is to be understood as encompassing contacting as well as mixing. Thus, treating the sample with the further enzyme may comprise any of mixing the sample with the enzyme, where the further enzyme may be free or immobilized on a carrier, passing the sample along a surface on which the further enzyme is immobilized, etc.

The antimicrobial substance is any antimicrobial substance that exerts an antimicrobial effect. It is understood that the antimicrobial substance in active form, i.e., the antimicrobial substance is present in the sample in a form, e.g., its normal, functional form, in which it exerts an antimicrobial effect on microbes. The antimicrobial substance is further understood to encompass antimicrobial substances that can be enzymatically inactivated. Such enzymatic inactivation may e.g. involve hydrolysis, transfer of functional groups or redox reactions.

Typically, the antimicrobial substance is an antibiotic. An antibiotic is any substance that kills bacteria. Preferably, the antibiotic is a beta-lactam antibiotic. Beta-lactam antibiotics are so called "broad spectrum" antibiotics and are among the most widely used group of antibiotics. The antimicrobial substance to be inactivated may thus for example be a beta-lactam. Beta-lactams are mainly active against Gram-positive bacteria, although beta-lactam antibiotics active against Gram-negative bacteria have also been developed. Preferably the beta-lactam antibiotic is a penicillin, Cephalosporin, cephamycin, monobactam, carbapenem or carbacephems. More preferably, the beta lactam antibiotic is penicillin, cephalosporin or carbapenem.

The further enzyme may be a beta-lactamase, preferably a carbapenemase, more preferably a class B carbapenemase. Beta-lactamases are a group of enzymes that provide bacterial resistance to beta-lactam antibiotics by breaking the antibiotic's structure by hydrolysis. The beta-lactamases used according to the technology proposed herein are preferably recombinantly produced. Carbapenemases are members of the molecular class A, B, or D β-lactamases. Class A and D enzymes have a serine-based hydrolytic mechanism, while class B enzymes are metallo-β-lactamases containing zinc in their active site. Carbapenemases can inactivate not only carbapenems but e.g also other broad-spectrum antibiotics such as penicillins, oxymino-cephalosporins, and cephamycins and are thus suitable to use as the inactivating enzyme according to the technology proposed herein as they can inactivate a wide range of antibiotics.

The class B carbapenemase may preferably be a New Delhi metallo-beta-lactamase (NDM). There are several variants of NDM, with NDM-1 being the most spread across different hosts. The variants have small genetic differences across them ranging between 1 to 5 amino acid substitutions compared to NDM-1 (maximum % variation is 5/270 amino acids)

Even more preferably, the further enzyme may be a New Delhi metallo-beta-lactamase 1 (NDM-1) enzyme. Corresponding polypeptides are given by DEQ ID Nos: 27, 28, 29, 30. The NDM-1 enzymes confer resistance to all β-lactams except aztreonam and are therefore an attractive choice as it will inactivate the majority of beta-lactam antibiotics by cleaving the beta-lactam ring of such antibiotics.

Preferably the further enzyme is a New Delhi metallo-beta-lactamase 1 (NDM-1) polypeptide comprising or consisting of a further polypeptide having at least 95% sequence identity to SEQ ID NO: 27 or 28, more preferably having at least 95% sequence identity to SEQ ID NO: 28.

The further enzyme may be a New Delhi metallo-beta-lactamase 1 (NDM-1) polypeptide comprising or consisting of a further polypeptide having at least 95%, such as at least 96%, such as at least 97%, such as at least 98%, such as at least 99%, such as 100%, sequence identity to SEQ ID NO: 27 or 28.

SEQ ID NO: 28 comprises an amino acid sequence for an NDM-1 protein, such as the NDM-1 protein of SEQ ID NO: 27 or a variant thereof as described elsewhere herein, preceded by a PelB leader sequence, a His-tag and a TEV protease recognition site on the N-terminal side of the NDM-1 amino acid sequence. The PelB leader sequence has the function of ensuring that the produced peptide is directed to the bacterial periplasm. Once in the periplasm, the PelB leader sequence is removed by a signal peptidase. The function of the His-tag is to allow the protein to be purified by affinity chromatography. The function of the TEV protease recognition site is to allow the His-tag to be cleaved off, rendering the final NDM-1 protein according to SEQ ID NO: 29. This polypeptide is therefore optimized for recombinant expression and purification of an NDM-1 protein.

Typically step (ii) comprises treating the sample with the further enzyme from 10 minutes to 40 minutes. The concentration of the further enzyme may be adjusted depending on the specific enzyme used. Typically, nanomolar to micromolar concentrations of the further enzyme is used, such as from about 10 nM to about 1 µM, such as from about 100 nM to about 500 nM, such as from about 100 nM to about 300 nM, such as about 200 nM (0.2 µM), in particular when NDM-1 is used as the further enzyme.

As above, a second aspect of the proposed technology relates to a method of obtaining a measurement of immunological activity of a sample, comprising the steps of:
i. performing the method according to the first aspect of the proposed technology, and
ii. obtaining a measurement of immunological activity of the sample against a microbe.

This is advantageous in that it provides for robustly obtaining a measurement of immunological activity of the sample.

Step (ii) is to be performed after step (i). Step (ii) of the method according to the second aspect of the proposed technology may alternatively be considered a third step of the method according to the first aspect of the proposed technology.

The term "immunological activity", as described herein, encompasses any feature, property or characteristic of the sample that influences immune cell activity. In particular, immunological activity refers to the extent that the sample influences and/or activates the human immune system such that the human immune system kills the microbe and/or protects the human body from the microbe.

Influencing and/or activating the human immune system may preferably encompass one or more of activating and/or facilitating opsonophagocytic killing of the microbe and activating and/or facilitating cytokine secretion. Corresponding assays for obtaining these measurements of immunological activity include Opsonophagocytic killing assay (OPkA) and complement activation assay.

It is further contemplated that Influencing and/or activating the human immune system may encompass one or more of activating and/or facilitating complement secretion or activation, and activating or facilitating monocyte ROS production. Corresponding assays for obtaining these measurements include cytokine secretion assay and monocyte oxidative burst assay.

Preferably the measurement of immunological activity comprises measuring the immunological activity of IgG antibodies in the sample. The measurement of immunological activity may thus comprise an amount or concentration or other measurement, such as a titer, of IgG antibodies against the microbe in the sample.

Preferably the microbe is a GBS bacteria, more preferably the GBS bacteria belongs to a GBS strain expressing at least one of the GBS surface proteins Rib and Alp2, most preferably the GBS bacteria belongs to Rib/CPSIII.

The GBS may be a GBS expressing any Alp type surface protein, such as any of Rib, AlpC, Alp1, Alp2, Alp3, and Alp4 protein, together with any CPS out of Ia Ib, II to X. Preferably the CPS is CPSIII or CPSV.
CPSIII refers to type III capsular polysaccharide.
CPSV refers to type V capsular polysaccharide.
The methods according to the various aspects of the proposed technology are especially suited to treating samples and obtaining measurements of immunological activity of the samples against GBS expressing either CPSIII or CPSV.

Preferably the measurement is obtained using an Opsonophagocytic killing Assay (OPkA). Accordingly, the immunological activity preferably encompasses opsonophagocytic killing activity.

Generally, opsonophagocytosis is the engulfment, by macrophages and other phagocytic cells like neutrophils, of bacteria opsonized with antibodies and/or complement proteins. Thus, microbial cells in the blood or within a specific tissue that become coated with IgG are soon recognized by patrolling macrophages and neutrophils. Typically, recognition occurs through specific surface-expressed FcγRs on the phagocytes, but the effect may also involve complement receptors. When multiple FcγRs are engaged simultaneously, it cues the phagocyte to envelope its prey and confine it within an intracellular compartment known as the phagosome. The phagosome is a dead-end for most microbial pathogens. Within minutes, the encased bacteria are exposed to reactive oxygen species, acidic pH and a barrage of degradative enzymes.

In an OPkA, phagocytic cells are co-cultured with bacterial cells and complement (e.g. baby rabbit complement). The phagocytic immune cells will phagocytose and kill the bacterial cells in a complement-dependent manner and the assay can be used to study the ability of such phagocytic immune cells to respond to and kill bacterial cells under different conditions. In particular, the OPkA can be used to measure the functionality of antibodies and for example assess protective immunity of Group B Streptococcus (GBS) vaccines. The efficiency of vaccines can thus be studied using an OPkA. In particular, the OPkA analysis can determine whether vaccination with a vaccine, e.g., a GBS vaccine candidate, results in the vaccinated subjects obtaining opsonizing antibodies, i.e., antibodies which when contacted with bacteria, bind to the bacteria and induce phagocytic cells to phagocytose the bacteria.

The OPkA is preferably configured for assaying the extent to which antibodies can mediate bacterial engulfment by immune cells. Such an OPkA preferably involves the binding of IgG to the GBS bacteria leading to a cascade of complement activation, resulting in deposition of C3b on the bacterial surface. This leads to phagocytosis by neutrophils through recognition of C3b by complement receptors on the neutrophils.

The measurement may comprise determining a single value representing a measure of immunological activity of the sample against a microbe. Alternatively, the measurement may comprise determining multiple values, or a distribution of values representing a measure of immunological activity of the sample against a microbe.

As above, a third aspect of the proposed technology relates to a method of obtaining a surrogate endpoint, comprising the steps of:
i. performing the method according to the second aspect of the proposed technology to obtain a measurement of immunological activity against a microbe for at least one sample associated with at least one of:
   a. a first group of infants not afflicted by disease and/or infection after having been exposed to a microbe and
   b. a second group of infants afflicted by disease and/or infection after being exposed to a microbe, and
ii. determining at least one surrogate endpoint based on the measurement.

This is advantageous in that a surrogate endpoint may be used to obtain a surrogate efficacy test.

Preferably a measurement is obtained for at least a plurality of samples. The plurality of samples may be at least 2, more preferably at least 8, such as at least 12. Accordingly, a measurement is preferably obtained for samples from at least 2, preferably at least 8, such as at least 12 infants. Accordingly, the at least one surrogate endpoint is preferably determined based on a plurality of measurements.

Preferably the sample is associated with the first group as this allows a surrogate endpoint corresponding to a protective immunity to the microbe to be determined. More preferably, measurements of immunological activity are obtained for samples associated with both infants of the first group and infants of the second group as this provides more robust surrogate endpoints associable with protective immunity on one hand, and no protective immunity on the other hand.

The surrogate endpoint may comprise a value. Thus, the surrogate endpoint may be a quantitative measurement of immunological activity. Alternatively, or additionally, the surrogate endpoint may comprise a distribution of measurements of immunological activity, i.e. comprise all or part of the full distribution of immune, or antibody, levels in the first and/or second group of infants. The at least one surrogate endpoint is based on the plurality of measurements. Expressed differently, the at least one surrogate endpoint is determined in relation to the plurality of measurements. The at least one surrogate endpoint may thus be based on a mean value, a minimum value, or a maximum value of the plurality of measurements. Additionally, the at least one surrogate endpoint may be based on further considerations, such as a safety margin or safety factor, applied in relation to the plurality of measurements, the distribution of measurements, and/or the mean, minimum, and/or maximum value of the plurality of measurements.

Typically, the surrogate endpoint, as a value, threshold, distribution of values, or statistical model, is obtained by statistical modelling so as to encompass a probability, i.e. 70%, 80% or 90%, that protection against infection has been obtained.

As above, a fourth aspect of the proposed technology relates to a surrogate efficacy test method for testing efficacy of a vaccine comprising the steps of:
i. providing at least one surrogate endpoint by performing the method according to the third aspect of the proposed technology,
ii. vaccinating at least one human using a vaccine against the microbe,
iii. performing the method according to the second aspect of the proposed technology on a sample from the at least one human or from the offspring of the at least one human, and
iv. comparing the measurement obtained with the at least one surrogate endpoint.

When the surrogate endpoint corresponds to protective immunity, then step (iv) of comparing the measurement obtained with the surrogate endpoint may comprise determining whether the measurement is statistically indistinguishable from the surrogate endpoint or not.

When the surrogate endpoint corresponds to no protective immunity, then step (iv) of comparing the measurement obtained with the surrogate endpoint may comprise determining whether the measurement is significantly distinguishable from the surrogate endpoint or not.

The vaccine may comprise the immunogenic fusion protein disclosed in WO2008/127179 or WO 2022/207657, alone or in combination with the immunogenic fusion protein disclosed in WO2017/068112. More particularly, the vaccine may comprise a combination of an immunogenic fusion protein comprising the N terminals of the Rib and AlpC GBS surface proteins, see SEQ ID NO: 31, combined with a further immunogenic fusion protein comprising the N terminals of the Alp1 and Alp2 GBS surface proteins, see SEQ ID NO: 32.

More preferably, the vaccine comprises an immunogenic fusion protein consisting of SEQ ID NO: 31 combined with a further immunogenic fusion protein consisting of SEQ ID NO: 32 in a buffer further comprising the adjuvant aluminum hydroxide.

However, the vaccine may be any vaccine based on, in other words comprising as antigen, a N-terminal of a GBS surface protein, e.g. Rib, AlpC, Alp1, Alp2, Alp3, Alp4, and/or a GBS capsular polysaccharide (CPS.

The human may be a male, a female, and adult, or a child. Preferably the human is a female, more preferably a pregnant female. More preferably the sample is from the offspring of the at least one human, such as from an infant born from the at least one human.

Most preferably, the at least one human vaccinated using the vaccine in step (ii) is a pregnant female and step (iii) of performing the method according to the second aspect of the proposed technology is performed on a sample from a human infant, or associated human umbilical cord, born from the pregnant female. This allows determining whether the vaccination of the pregnant female provides sufficient protection from the microbe to the infant born from the pregnant female.

The sample obtained in step (iii) is preferably the same type of sample as used to obtain the surrogate endpoint by performing the method according to the third aspect in step (i). Type here refers to the type of sample (blood, serum, plasma) and the source of sample (human infant, human umbilical cord). Depending on the surrogate endpoint, i.e. whether the surrogate endpoint comprises a single value or a distribution of values, the comparison in step (iv) may comprise comparing a single value to the surrogate endpoint or a distribution of measurements, to the surrogate endpoint.

As above, a fifth aspect of the proposed technology relates to a use of an enzyme as defined herein to inactivate and/or cleave human IgM in a sample comprising blood, serum, and/or plasma from a human infant or from a human umbilical cord.

The above discussion of the first aspect of the proposed technology also applies to the fifth aspect of the proposed technology.

As above, a sixth aspect of the proposed technology relates to a kit comprising:
- an enzyme as defined herein, and
- a further enzyme as defined herein

The kit is advantageous in that it allows treating samples using both the enzyme and the further enzyme to prevent or reduce errors in a later analysis of the sample.

According to the technology proposed herein, sequence identity is calculated in the following manner. By an amino acid sequence having a sequence having at least, for example at least 95%, sequence identity to a reference sequence, is intended that the sequence is identical to the reference sequence except that the sequence may include up to 5 amino acid differences per each 100 amino acids of the reference sequence. In other words, to obtain an amino acid sequence having a sequence identity of at least 95%, up to 5% of the amino acids in the reference sequence may be deleted or substituted with another amino acid, or a number of amino acids up to 5% of the total number of amino acids in the reference sequence may be inserted into the reference sequence. If the two sequences to be compared are not of equal length, they must be aligned to best possible fit, and the sequence identity calculated over the length of the reference sequence. These amino acid changes of the reference sequence may occur at the amino and/or carboxy terminal positions of a reference amino acid sequence or anywhere between those terminal positions, interspersed either individually within the reference sequence or in one or more contiguous groups within the reference sequence.

Table 1 below details the sequences mentioned herein:

**Table 1: sequences**

| Sequence ID / comment | Sequence |
|---|---|
| SEQ ID NO: 1 | NIVESDQDKANRIKSSLTTVKNDNVVQYTNEDGEIRTIIWAKGINGPT MGEGGDFHKEITNAGSDTYIDYKTSFTTGDGWYDVNKTRAGGNV DVDKNLCFGAVSSNMLHWWFEQNIEYIDRYIVKNGDIIRANRKISDL KYSFKSQQNSAIFELFKVLFGNNEKGFYTDLLMDLFINGYTPKTGG GTNIERDDLTPDSRGGFFYDVFKGEKLTDRTYGGSYEALGDKLKEV LGEAGLIGISHKAFKNSNHIVTLWGAEYDLNGKLIAVYISDSDDQNET EDERDLGMKRYEVRNVGGVAKLSTNQTNKSSGSAIGYLHILYLGTT QWENYFK |
| SEQ ID NO: 2 | MKKFNGKSVFLGLAAATIISSNAYAGIEGWKFEGNNWKYYKEDKAL KAWQQLNNKWYFFNEDGSLKTGWHLDATNNWYFLDSSKNSNEG MLLSGWQWIDGYCYYFENIDKSTLGRMYVNTVVNGYRIDATGRWT NEKGVEYYEAGKGIITVNTLQGSLNTGKKSSGGGSSGKGGGLGKS GESGKGGSSIGGIGGKTGINQGDNNDTSNQSLIVEENSTVNNADQ TKSVEKTKGDQKSEQNNSNEVNTTEGGQNKSGMTNNTSEYTYNK PTVNKENEDEKTNTTQDNKVSDSTSNDLSKTKDTQTNTNDSDIKSA TASNAEIQKEEEHKNTETTNSSEKNKNTEQQNKSEENIVESDQDKA NRIKSSLTTVKNDNVVQYTNEDGEIRTIIWAKGINGPTMGEGGDFHK EITNAGSDTYIDYKTSFTTGDGWYDVNKTRAGGNVDVDKNLCFGA VSSNMLHWWFEQNIEYIDRYIVKNGDIIRANRKISDLKYSFKSQQNS AIFELFKVLFGNNEKGFYTDLLMDLFINGYTPKTGGGTNIERDDLTP DSRGGFFYDVFKGEKLTDRTYGGSYEALGDKLKEVLGEAGLIGISH KAFKNSNHIVTLWGAEYDLNGKLIAVYISDSDDQNETEDERDLGMK RYEVRNVGGVAKLSTNQTNKSSGSAIGYLHILYLGTTQWENYFK |
| SEQ ID NO: 3 | MGIEGWKFEGNNWKYYKEDKALKAWQQLNNKWYFFNEDGSLKT GWHLDATNNWYFLDSSKNSNEGMLLSGWQWIDGYCYYFENIDKS |
| | TLGRMYVNTVVNGYRIDATGRWTNEKGVEYYEAGKGIITVNTLQGS LNTGKKSSGGGSSGKGGGLGKSGESGKGGSSIGGIGGKTGINQG DNNDTSNQSLIVEENSTVNNADQTKSVEKTKGDQKSEQNNSNEVN TTEGGQNKSGMTNNTSEYTYNKPTVNKENEDEKTNTTQDNKVSD STSNDLSKTKDTQTNTNDSDIKSATASNAEIQKEEEHKNTETTNSSE KNKNTEQQNKSEENIVESDQDKANRIKSSLTTVKNDNVVQYTNED GEIRTIIWAKGINGPTMGEGGDFHKEITNAGSDTYIDYKTSFTTGDG WYDVNKTRAGGNVDVDKNLCFGAVSSNMLHWWFEQNIEYIDRYIV KNGDIIRANRKISDLKYSFKSQQNSAIFELFKVLFGNNEKGFYTDLL MDLFINGYTPKTGGGTNIERDDLTPDSRGGFFYDVFKGEKLTDRTY GGSYEALGDKLKEVLGEAGLIGISHKAFKNSNHIVTLWGAEYDLNG KLIAVYISDSDDQNETEDERDLGMKRYEVRNVGGVAKLSTNQTNKS SGSAIGYLHILYLGTTQWENYFKHHHHHH |
| SEQ ID NO: 4 | MHHHHHHHHHGIEGWKFEGNNWKYYKEDKALKAWQQLNNKWY FFNEDGSLKTGWHLDATNNWYFLDSSKNSNEGMLLSGWQWIDGY CYYFENIDKSTLGRMYVNTVVNGYRIDATGRWTNEKGVEYYEAGK GIITVNTLQGSLNTGKKSSGGGSSGKGGGLGKSGESGKGGSSIGG IGGKTGINQGDNNDTSNQSLIVEENSTVNNADQTKSVEKTKGDQK SEQNNSNEVNTTEGGQNKSGMTNNTSEYTYNKPTVNKENEDEKT NTTQDNKVSDSTSNDLSKTKDTQTNTNDSDIKSATASNAEIQKEEE HKNTETTNSSEKNKNTEQQNKSEENIVESDQDKANRIKSSLTTVKN DNVVQYTNEDGEIRTIIWAKGINGPTMGEGGDFHKEITNAGSDTYID YKTSFTTGDGWYDVNKTRAGGNVDVDKNLCFGAVSSNMLHWWF EQNIEYIDRYIVKNGDIIRANRKISDLKYSFKSQQNSAIFELFKVLFGN NEKGFYTDLLMDLFINGYTPKTGGGTNIERDDLTPDSRGGFFYDVF KGEKLTDRTYGGSYEALGDKLKEVLGEAGLIGISHKAFKNSNHIVTL WGAEYDLNGKLIAVYISDSDDQNETEDERDLGMKRYEVRNVGGVA KLSTNQTNKSSGSAIGYLHILYLGTTQWENYFK |
| SEQ ID NO: 5 | MQNKSGMTNNTSEYTYNKPTVNKENEDEKTNTTQDNKVSDSTSN DLSKTKDTQTNTNDSDIKSATASNAEIQKEEEHKNTETTNSSEKNK NTEQQNKSEENIVESDQDKANRIKSSLTTVKNDNVVQYTNEDGEIR TIIWAKGINGPTMGEGGDFHKEITNAGSDTYIDYKTSFTTGDGWYD VNKTRAGGNVDVDKNLCFGAVSSNMLHWWFEQNIEYIDRYIVKNG DIIRANRKISDLKYSFKSQQNSAIFELFKVLFGNNEKGFYTDLLMDLF INGYTPKTGGGTNIERDDLTPDSRGGFFYDVFKGEKLTDRTYGGSY EALGDKLKEVLGEAGLIGISHKAFKNSNHIVTLWGAEYDLNGKLIAV YISDSDDQNETEDERDLGMKRYEVRNVGGVAKLSTNQTNKSSGSA IGYLHILYLGTTQWENYFKHHHHHH |
| SEQ ID NO: 6 | MSDSTSNDLSKTKDTQTNTNDSDIKSATASNAEIQKEEEHKNTETT NSSEKNKNTEQQNKSEENIVESDQDKANRIKSSLTTVKNDNVVQYT NEDGEIRTIIWAKGINGPTMGEGGDFHKEITNAGSDTYIDYKTSFTT GDGWYDVNKTRAGGNVDVDKNLCFGAVSSNMLHWWFEQNIEYID RYIVKNGDIIRANRKISDLKYSFKSQQNSAIFELFKVLFGNNEKGFYT DLLMDLFINGYTPKTGGGTNIERDDLTPDSRGGFFYDVFKGEKLTD RTYGGSYEALGDKLKEVLGEAGLIGISHKAFKNSNHIVTLWGAEYDL NGKLIAVYISDSDDQNETEDERDLGMKRYEVRNVGGVAKLSTNQT NKSSGSAIGYLHILYLGTTQWENYFKLEHHHHHH |
| SEQ ID NO: 7 | MNIVESDQDKANRIKSSLTTVKNDNVVQYTNEDGEIRTIIWAKGING PTMGEGGDFHKEITNAGSDTYIDYKTSFTTGDGWYDVNKTRAGGN VDVDKNLCFGAVSSNMLHWWFEQNIEYIDRYIVKNGDIIRANRKISD LKYSFKSQQNSAIFELFKVLFGNNEKGFYTDLLMDLFINGYTPKTGG GTNIERDDLTPDSRGGFFYDVFKGEKLTDRTYGGSYEALGDKLKEV LGEAGLIGISHKAFKNSNHIVTLWGAEYDLNGKLIAVYISDSDDQNET EDERDLGMKRYEVRNVGGVAKLSTNQTNKSSGSAIGYLHILYLGTT QWENYFKHHHHHH |
| SEQ ID NO: 8 | AERKESTQNRAERFKESLITSENDNVVQYTNEDGEIRTIIWVKGING PVMGEGGDFHKEITQGGKNTYVMYTVPYSSGDGWYDVNKTRSG GNIDIDKNLCFAAVSSNMLHWWFDQNIEYVDRYIEKNGDIIRANRKL SDLKTSFENPENSKIFELYKVLYGYNERGFYSDLLMDLFINGYTPNP KGGTNIESDDLIPNNNGGFFYDVFKGKKLTDRTYGGNYESLSNLLK EILGNGGIVGLSHKVFSKSNHIVTLWGAEYDLNGKLKAVYISDSDDQ DESNVGMKRFEIRNVGGKAKISTNETDKSAGAELGYLHILYQGTGQ WENYFK |
| SEQ ID NO: 9 | MKKYKVGPVLLGLTMLMSFNAYAGVDGWKQENNSWKYYKEDKAL KAWQQINNNWYFFNEDGSLKTGWYMDAANNWYFLDSSKTANEG VLLSGWQWIDGYCYYFEGIDNNTFGKMYSNTLVSGYKVDALGRWI NESGAEYYEAGKGISTGNASQSSSNTAKKTSVAGSSEKSGKSGGS SKGGSSGKSGGSSKGGSSGKSGGSSFLNQGEKREENRILGESKG KAENSSSKRSEDLVGSRQDLSSDKNNSLIADNKTGNSEKAGSIEKN TNKEYTTKSNESPVKPATSSNVEIPKEEEKKDIESKESKKPEAERKE STQNRAERFKESLITSENDNVVQYTNEDGEIRTIIWVKGINGPVMGE GGDFHKEITQGGKNTYVMYTVPYSSGDGWYDVNKTRSGGNIDIDK NLCFAAVSSNMLHWWFDQNIEYVDRYIEKNGDIIRANRKLSDLKTSF ENPENSKIFELYKVLYGYNERGFYSDLLMDLFINGYTPNPKGGTNIE SDDLIPNNNGGFFYDVFKGKKLTDRTYGGNYESLSNLLKEILGNGGI VGLSHKVFSKSNHIVTLWGAEYDLNGKLKAVYISDSDDQDESNVG MKRFEIRNVGGKAKISTNETDKSAGAELGYLHILYQGTGQWENYFK |
| SEQ ID NO: 10 | MGVDGWKQENNSWKYYKEDKALKAWQQINNNWYFFNEDGSLKT GWYMDAANNWYFLDSSKTANEGVLLSGWQWIDGYCYYFEGIDNN TFGKMYSNTLVSGYKVDALGRWINESGAEYYEAGKGISTGNASQS SSNTAKKTSVAGSSEKSGKSGGSSKGGSSGKSGGSSKGGSSGKS GGSSFLNQGEKREENRILGESKGKAENSSSKRSEDLVGSRQDLSS DKNNSLIADNKTGNSEKAGSIEKNTNKEYTTKSNESPVKPATSSNV EIPKEEEKKDIESKESKKPEAERKESTQNRAERFKESLITSENDNVV QYTNEDGEIRTIIWVKGINGPVMGEGGDFHKEITQGGKNTYVMYTV PYSSGDGWYDVNKTRSGGNIDIDKNLCFAAVSSNMLHWWFDQNIE YVDRYIEKNGDIIRANRKLSDLKTSFENPENSKIFELYKVLYGYNERG FYSDLLMDLFINGYTPNPKGGTNIESDDLIPNNNGGFFYDVFKGKKL TDRTYGGNYESLSNLLKEILGNGGIVGLSHKVFSKSNHIVTLWGAEY DLNGKLKAVYISDSDDQDESNVGMKRFEIRNVGGKAKISTNETDKS AGAELGYLHILYQGTGQWENYFKHHHHHH |
| SEQ ID NO: 11 | MHHHHHHHHHGVDGWKQENNSWKYYKEDKALKAWQQINNNW YFFNEDGSLKTGWYMDAANNWYFLDSSKTANEGVLLSGWQWIDG YCYYFEGIDNNTFGKMYSNTLVSGYKVDALGRWINESGAEYYEAG KGISTGNASQSSSNTAKKTSVAGSSEKSGKSGGSSKGGSSGKSG GSSKGGSSGKSGGSSFLNQGEKREENRILGESKGKAENSSSKRS EDLVGSRQDLSSDKNNSLIADNKTGNSEKAGSIEKNTNKEYTTKSN ESPVKPATSSNVEIPKEEEKKDIESKESKKPEAERKESTQNRAERF KESLITSENDNVVQYTNEDGEIRTIIWVKGINGPVMGEGGDFHKEIT QGGKNTYVMYTVPYSSGDGWYDVNKTRSGGNIDIDKNLCFAAVSS NMLHWWFDQNIEYVDRYIEKNGDIIRANRKLSDLKTSFENPENSKIF ELYKVLYGYNERGFYSDLLMDLFINGYTPNPKGGTNIESDDLIPNNN GGFFYDVFKGKKLTDRTYGGNYESLSNLLKEILGNGGIVGLSHKVF SKSNHIVTLWGAEYDLNGKLKAVYISDSDDQDESNVGMKRFEIRNV GGKAKISTNETDKSAGAELGYLHILYQGTGQWENYFK |
| SEQ ID NO: 12 Encodes SEQ ID NO: 1 | aatatagtagagagtgatcaggataaggccaatagaataaaatcctctttgactaccgttaaaa atgataatgtggtacagtacacgaatgaagatggagaaattagaacaattatttgggcaaagg gcatcaatggacctactatgggagagggtggagattttcacaaagaaataacaaatgcgggtt cagatacatatatagattataaaacttcgttcactacaggtgatggatggtatgatgtaaataag acaagagccggtggcaatgtagacgtagataaaaatctttgctttggggcagtttcatccaatat gctacattggtggtttgaacaaaatattgaatatattgacagatatatagtaaagaatggagatat aattcgtgcaaataggaagatctcagatctgaaatattcttttaagagtcagcaaaatagtgctat attcgaactctttaaggtgttatttggaaataatgagaagggattttatacagaccttttgatggacc tgtttataaacggatatactccaaagacaggtggaggtacaaatatagaaagagatgatttaa cacctgatagcagaggtggatttttctatgatgtgtttaaaggagagaagctgacagaccgtact tatggtggaagttatgaagctttaggtgataaattaaaagaagtactgggagaagccggtttgat aggtatatctcataaggcttttaaaaacagtaatcatatagtaactctatggggtgctgaatatgat ttgaatggaaagcttatagcggtatatattagtgattcagatgatcaaaatgaaacagaagatg aaagagatctgggcatgaaacgctatgaggttaggaatgtaggaggagtggcaaaactgtcc acaaatcaaacgaataagtcgtcaggttcagctataggatatcttcatatactatacttaggaac cacacagtgggaaaattattttaaataa |
| SEQ ID NO: 13 | atgaaaaagtttaacggtaagtctgtttttttaggacttgcagcagcaactataatctcatctaatg cctatgcaggtattgaaggatggaaatttgagggtaataattggaaatattataaagaagataa agctttaaaggcctggcagcaattaaataataaatggtattttttaatgaggatggaagtttaaa aacaggatggcatttggatgctacaaataattggtactttttagacagctctaaaaattctaatga aggtatgcttttgtcaggatggcagtggatagacggatactgctattattttgaaaacattgataa atctactcttggcagaatgtatgtaaatactgtagtgaatggatatagaattgatgctacaggtag atggacaaatgaaaaaggtgttgaatattatgaagcaggcaagggaataattacagttaatac tttacaaggctctttaaatacaggtaaaaaatcaagtggaggtggaagctccggtaaaggtggt ggtttgggaaaaagcggagaatccggtaaaggtggatcaagtatagggggtattggtggaaa aaccggcataaatcaaggcgataataatgatacttcaaatcaaagcctgatagtagaagaaa attcgactgtgaataatgcagatcagactaagagtgtagaaaaaacaaagggtgatcaaaa atctgaacaaaataattcaaatgaagtaaatactactgaaggtggtcagaacaaatccggaat gactaataatacaagtgagtatacttataataaacctactgtgaataaagaaaatgaagatga aaaaactaatacaacacaggataacaaagtatcggatagcacttcaaatgatttgagtaaaa caaaagatacacagacaaatacaaatgatagtgatataaagtcggctactgcttcaaatgcc gaaatccaaaaggaagaagaacataaaaatacagaaactacgaatagttctgagaagaat aaaaatactgaacagcagaacaaatctgaagagaatatagtagagagtgatcaggataag gccaatagaataaaatcctctttgactaccgttaaaaatgataatgtggtacagtacacgaatg aagatggagaaattagaacaattatttggcaaagggcatcaatggacctactatgggagag ggtggagattttcacaaagaaataacaaatgcgggttcagatacatatatagattataaacttc gttcactacaggtgatggatggtatgatgtaaataagacaagagccggtggcaatgtagacgt agataaaaatctttgctttggggcagtttcatccaatatgctacattggtggtttgaacaaaatattg aatatattgacagatatatagtaaagaatggagatataattcgtgcaaataggaagatctcaga tctgaaatattcttttaagagtcagcaaaatagtgctatattcgaactctttaaggtgttatttggaaa taatgagaagggattttatacagaccttttgatggacctgtttataaacggatatactccaaagac aggtggaggtacaaatatagaaagagatgatttaacacctgatagcagaggtggatttttctat gatgtgtttaaaggagagaagctgacagaccgtacttatggtggaagttatgaagctttaggtg ataaattaaaagaagtactgggagaagccggtttgataggtatatctcataaggcttttaaaaa cagtaatcatatagtaactctatggggtgctgaatatgatttgaatggaaagcttatagcggtata tattagtgattcagatgatcaaaatgaaacagaagatgaaagagatctgggcatgaaacgct atgaggttaggaatgtaggaggagtggcaaaactgtccacaaatcaaacgaataagtcgtca ggttcagctataggatatcttcatatactatacttaggaaccacacagtgggaaaattattttaaat aa |
| Encodes SEQ ID NO: 2 | |
| SEQ ID NO: 14 | atgggcatcgaaggttggaaattcgagggtaacaactggaagtactataaagaggataagg cgctgaaagcgtggcagcaactgaacaacaaatggtacttctttaacgaagacggcagcctg aagaccggttggcacctggatgcgaccaacaactggtatttcctggacagcagcaaaaaca gcaacgagggcatgctgctgagcggttggcaatggatcgatggttactgctactacttcgaaaa catcgacaagagcaccctgggccgtatgtacgttaacaccgtggttaacggctatcgtatcgac gcgaccggtcgttggaccaacgagaaaggcgttgaatactatgaggcgggcaagggtatca ttaccgtgaacaccctgcagggcagcctgaacaccggtaagaaaagcagcggtggcggta gcagcggcaagggcggtggcctgggcaaaagcggtgaaagcggcaagggtggcagcag catcggtggcattggtggcaaaaccggcatcaaccagggtgacaacaacgataccagcaa ccaaagcctgattgttgaggaaaacagcaccgtgaacaacgcggatcaaaccaaaagcgtt gagaagaccaaaggtgaccagaagagcgaacaaaacaacagcaacgaagtgaacacc |
| Encodes SEQ ID NO: 3 | |
| | accgagggtggccagaacaagagcggcatgaccaacaacaccagcgagtacacctataa caagccgaccgttaacaaagaaaacgaggatgaaaaaccaacaccacccaggacaac aaagtgagcgatagcaccagcaacgacctgagcaagaccaaagacacccaaaccaaca ccaacgacagcgatatcaaaagcgcgaccgcgagcaacgcggagattcagaaggaaga ggagcacaaaaacaccgaaaccaccaacagcagcgagaagaacaaaaacaccgaac agcaaaacaagagcgaagagaacatcgttgaaagcgaccaagataaggcgaaccgtatt aaaagcagcctgaccaccgtgaaaaacgataacgtggttcagtacaccaacgaagacggc gagatccgtaccatcatttgggcgaagggtattaacggcccgaccatgggcgagggtggcga tttccacaaagaaatcaccaacgcgggcagcgacacctacattgattataagaccagctttac caccggcgacggttggtatgatgttaacaaaacccgtgcgggtggcaacgtggacgttgataa gaacctgtgcttcggtgctgtgagcagcaacatgctgcactggtggtttgaacagaacatcgag tacattgatcgttatatcgttaaaaacggcgacatcattcgtgcgaaccgtaagattagcgacct gaagtacagcttcaaaagccagcaaaacagcgcgatcttcgagctgtttaaagtgctgttcgg caacaacgaaaagggtttttacaccgacctgctgatggacctgttcatcaacggttataccccg aaaaccggtggcggtaccaacattgagcgtgacgatctgaccccggatagccgtggcggttt cttttacgacgttttcaagggcgagaaactgaccgatcgtacctacggcggtagctatgaagcg ctgggcgacaagctgaaagaagtgctgggcgaggcgggcctgatcggtattagccacaagg cgtttaaaaatagcaaccacatcgttaccctgtggggtgcggagtacgatctgaacggcaaac tgatcgcggtgtatattagcgacagcgacgatcagaacgagaccgaagacgagcgtgatctg ggtatgaagcgttacgaagtgcgtaacgttggcggtgtggcgaaactgagcaccaaccaaac caacaagagcagcggcagcgcgatcggttacctgcacattctgtatctgggtaccacccagtg ggaaaactatttcaagctcgagcaccaccaccaccaccactga |
| SEQ ID NO: 15 | atgcaccaccaccaccaccaccaccaccaccacggcatcgaaggttggaaattcgagggta acaactggaagtactataaagaggataaggcgctgaaagcgtggcagcaactgaacaaca aatggtacttctttaacgaagacggcagcctgaagaccggttggcacctggatgcgaccaac aactggtatttcctggacagcagcaaaaacagcaacgagggcatgctgctgagcggttggca atggatcgatggttactgctactacttcgaaaacatcgacaagagcaccctgggccgtatgtac gttaacaccgtggttaacggctatcgtatcgacgcgaccggtcgttggaccaacgagaaagg cgttgaatactatgaggcgggcaagggtatcattaccgtgaacaccctgcagggcagcctga acaccggtaagaaaagcagcggtggcggtagcagcggcaagggcggtggcctgggcaaa agcggtgaaagcggcaagggtggcagcagcatcggtggcattggtggcaaaaccggcatc aaccagggtgacaacaacgataccagcaaccaaagcctgattgttgaggaaaacagcacc gtgaacaacgcggatcaaaccaaaagcgttgagaagaccaaaggtgaccagaagagcg aacaaaacaacagcaacgaagtgaacaccaccgagggtggccagaacaagagcggcat gaccaacaacaccagcgagtacacctataacaagccgaccgttaacaaagaaaacgagg atgaaaaaccaacaccacccaggacaacaaagtgagcgatagcaccagcaacgacctg agcaagaccaaagacacccaaaccaacaccaacgacagcgatatcaaaagcgcgaccg cgagcaacgcggagattcagaaggaagaggagcacaaaaacaccgaaaccaccaaca gcagcgagaagaacaaaaacaccgaacagcaaaacaagagcgaagagaacatcgttg aaagcgaccaagataaggcgaaccgtattaaaagcagcctgaccaccgtgaaaaacgata acgtggttcagtacaccaacgaagacggcgagatccgtaccatcatttgggcgaagggtatta acggcccgaccatgggcgagggtggcgatttccacaaagaaatcaccaacgcgggcagcg acacctacattgattataagaccagctttaccaccggcgacggttggtatgatgttaacaaaac ccgtgcgggtggcaacgtggacgttgataagaacctgtgcttcggtgctgtgagcagcaacat gctgcactggtggtttgaacagaacatcgagtacattgatcgttatatcgttaaaaacggcgac atcattcgtgcgaaccgtaagattagcgacctgaagtacagcttcaaaagccagcaaaacag cgcgatcttcgagctgtttaaagtgctgttcggcaacaacgaaaagggtttttacaccgacctgc tgatggacctgttcatcaacggttataccccgaaaaccggtggcggtaccaacattgagcgtg acgatctgaccccggatagccgtggcggtttcttttacgacgttttcaagggcgagaaactgac cgatcgtacctacggcggtagctatgaagcgctgggcgacaagctgaaagaagtgctgggc gaggcgggcctgatcggtattagccacaaggcgtttaaaaatagcaaccacatcgttaccctg tggggtgcggagtacgatctgaacggcaaactgatcgcggtgtatattagcgacagcgacga tcagaacgagaccgaagacgagcgtgatctgggtatgaagcgttacgaagtgcgtaacgttg gcggtgtggcgaaactgagcaccaaccaaaccaacaagagcagcggcagcgcgatcggt tacctgcacattctgtatctgggtaccacccagtgggaaaactatttcaagctcgagtga |
| Encodes SEQ ID NO: 4 | |
| SEQ ID NO: 16 | atgcagaacaagagcggcatgaccaacaacaccagcgagtacacctataacaagccgac cgttaacaaagaaaacgaggatgaaaaaccaacaccacccaggacaacaaagtgagc gatagcaccagcaacgacctgagcaagaccaaagacacccaaaccaacaccaacgaca gcgatatcaaaagcgcgaccgcgagcaacgcggagattcagaaggaagaggagcacaa aaacaccgaaaccaccaacagcagcgagaagaacaaaaacaccgaacagcaaaaca agagcgaagagaacatcgttgaaagcgaccaagataaggcgaaccgtattaaaagcagc ctgaccaccgtgaaaaacgataacgtggttcagtacaccaacgaagacggcgagatccgta ccatcatttgggcgaagggtattaacggcccgaccatgggcgagggtggcgatttccacaaa gaaatcaccaacgcgggcagcgacacctacattgattataagaccagctttaccaccggcga cggttggtatgatgttaacaaaacccgtgcgggtggcaacgtggacgttgataagaacctgtg cttcggtgctgtgagcagcaacatgctgcactggtggtttgaacagaacatcgagtacattgatc gttatatcgttaaaaacggcgacatcattcgtgcgaaccgtaagattagcgacctgaagtacag cttcaaaagccagcaaaacagcgcgatcttcgagctgtttaaagtgctgttcggcaacaacga aaagggtttttacaccgacctgctgatggacctgttcatcaacggttataccccgaaaaccggt ggcggtaccaacattgagcgtgacgatctgaccccggatagccgtggcggtttcttttacgacg ttttcaagggcgagaaactgaccgatcgtacctacggcggtagctatgaagcgctgggcgac aagctgaaagaagtgctgggcgaggcgggcctgatcggtattagccacaaggcgtttaaaa atagcaaccacatcgttaccctgtggggtgcggagtacgatctgaacggcaaactgatcgcg gtgtatattagcgacagcgacgatcagaacgagaccgaagacgagcgtgatctgggtatga agcgttacgaagtgcgtaacgttggcggtgtggcgaaactgagcaccaaccaaaccaacaa gagcagcggcagcgcgatcggttacctgcacattctgtatctgggtaccacccagtgggaaa actatttcaagctcgagcaccaccaccaccaccactga |
| Encodes SEQ ID NO: 5 | |
| SEQ ID NO: 17 | atgagcgatagcaccagcaacgacctgagcaagaccaaagacacccaaaccaacacca acgacagcgatatcaaaagcgcgaccgcgagcaacgcggagattcagaaggaagagga gcacaaaaacaccgaaaccaccaacagcagcgagaagaacaaaaacaccgaacagca aaacaagagcgaagagaacatcgttgaaagcgaccaagataaggcgaaccgtattaaa gcagcctgaccaccgtgaaaaacgataacgtggttcagtacaccaacgaagacggcgaga tccgtaccatcatttgggcgaagggtattaacggcccgaccatgggcgagggtggcgatttcc acaaagaaatcaccaacgcgggcagcgacacctacattgattataagaccagctttaccacc ggcgacggttggtatgatgttaacaaaacccgtgcgggtggcaacgtggacgttgataagaa cctgtgcttcggtgctgtgagcagcaacatgctgcactggtggtttgaacagaacatcgagtac attgatcgttatatcgttaaaaacggcgacatcattcgtgcgaaccgtaagattagcgacctgaa gtacagcttcaaaagccagcaaaacagcgcgatcttcgagctgtttaaagtgctgttcggcaa caacgaaaagggtttttacaccgacctgctgatggacctgttcatcaacggttataccccgaaa accggtggcggtaccaacattgagcgtgacgatctgaccccggatagccgtggcggtttctttt acgacgttttcaagggcgagaaactgaccgatcgtacctacggcggtagctatgaagcgctg ggcgacaagctgaaagaagtgctgggcgaggcgggcctgatcggtattagccacaaggcgt ttaaaaatagcaaccacatcgttaccctgtggggtgcggagtacgatctgaacggcaaactga tcgcggtgtatattagcgacagcgacgatcagaacgagaccgaagacgagcgtgatctgggt atgaagcgttacgaagtgcgtaacgttggcggtgtggcgaaactgagcaccaaccaaacca acaagagcagcggcagcgcgatcggttacctgcacattctgtatctgggtaccacccagtggg aaaactatttcaagctcgagcaccaccaccaccaccactga |
| Encodes SEQ ID NO: 6 | |
| SEQ ID NO: 18 | atgaacatcgttgaaagcgaccaagataaggcgaaccgtattaaaagcagcctgaccaccg tgaaaaacgataacgtggttcagtacaccaacgaagacggcgagatccgtaccatcatttgg gcgaagggtattaacggcccgaccatgggcgagggtggcgatttccacaaagaaatcacca acgcgggcagcgacacctacattgattataagaccagctttaccaccggcgacggttggtatg atgttaacaaaacccgtgcgggtggcaacgtggacgttgataagaacctgtgcttcggtgctgt gagcagcaacatgctgcactggtggtttgaacagaacatcgagtacattgatcgttatatcgtta aaaacggcgacatcattcgtgcgaaccgtaagattagcgacctgaagtacagcttcaaaagc cagcaaaacagcgcgatcttcgagctgtttaaagtgctgttcggcaacaacgaaaagggttttt acaccgacctgctgatggacctgttcatcaacggttataccccgaaaaccggtggcggtacca acattgagcgtgacgatctgaccccggatagccgtggcggtttcttttacgacgttttcaagggc gagaaactgaccgatcgtacctacggcggtagctatgaagcgctgggcgacaagctgaaag aagtgctgggcgaggcgggcctgatcggtattagccacaaggcgtttaaaaatagcaaccac atcgttaccctgtggggtgcggagtacgatctgaacggcaaactgatcgcggtgtatattagcg |
| Encodes SEQ ID NO: 7 | |
| | acagcgacgatcagaacgagaccgaagacgagcgtgatctgggtatgaagcgttacgaag tgcgtaacgttggcggtgtggcgaaactgagcaccaaccaaaccaacaagagcagcggca gcgcgatcggttacctgcacattctgtatctgggtaccacccagtgggaaaactatttcaagctc gagcaccaccaccaccaccactga |
| SEQ ID NO: 19 | gcagaaagaaaagagagtacacaaaacagggctgagagatttaaagaatcattaattacttc agagaatgacaatgttgttcagtatacaaatgaagacggtgaaatacgaaccatcatatgggt aaagggaattaacggacctgttatgggagaaggtggagactttcataaagagattacacaag gcggtaagaatacttatgtaatgtatactgtaccatattcaagcggagacggatggtatgatgtta ataaaacaagaagcggtggaaatatcgatatagataaaaatctttgctttgcagcggtatcatc aaatatgcttcattggtggtttgatcaaaatattgaatatgttgacagatatatagaaaaaaacgg agatataattcgtgccaacagaaaactttcagatttaaaaacttcctttgaaaatccggaaaac agtaaaatatttgagctgtataaagtattgtacggatataatgagagagggttttattcggatctttt gatggatttatttattaacggatatactccaaacccaaagggtggaacaaatatagaaagtgat gatcttatcccaaataataacggcggattcttctatgatgttttcaagggggaaaaacttacaga ccgtacttatggtggaaactatgaatcactaagtaatcttcttaaagaaatcttgggtaatggcgg catagtaggactttctcataaagtgttttcaaagagtaatcatatagttactttgtggggtgccgaat atgacttgaacgggaagttgaaggctgtatatataagcgattctgacgatcaggatgaaagta atgtaggcatgaaacgttttgaaatccgaaatgtaggaggaaaagcaaaaatatctaccaac gaaacagataagtctgcgggtgctgaattaggatatcttcatatcctatatcagggaaccggac aatgggaaaattattttaaataa |
| Encodes SEQ ID NO: 8 | |
| SEQ ID NO: 20 | atgaaaaatataaagtcggtcctgtattgttgggacttactatgttgatgtcctttaatgcctatgc aggtgttgatggatggaaacaggaaaataacagttggaaatattataaagaagataaggctct aaaggcttggcagcagataaataataattggtatttctttaatgaagacggaagtttaaaaacc ggttggtatatggacgccgcaaataattggtatttttttagacagctctaaaactgccaatgaggg agtgcttttatcaggatggcagtggatagacggatattgctattattttgagggaatagataataat acattcggaaagatgtattcaaatactcttgtcagcggatataaagttgatgctttaggaagatgg attaatgaaagcggagcagaatattatgaagccggtaaaggtataagtacaggtaatgcttca caaagttcatcgaatacagcaaagaaaacaagtgttgcaggaagttcggaaaaaagcggta aaagcggaggctcaagcaagggcggaagctccggaaaaagcggaggctcaagcaagg gcggaagttccggtaaaagcggaggctcaagctttttaaatcaaggagaaaaaagagaaga aaacagaatattaggagaatcaaaaggaaaggctgaaaactcaagttccaaaagaagtga agacttggtaggttcaagacaagacttaagctcggataaaaataacagcttgattgcagataat aaaacaggaaatagtgagaaagccggaagcatagaaaaaaatacaaataaagagtatac cacaaagtcaaatgagagccctgtaaaaccggccacttcctcaaatgtcgagataccgaaa gaagaggaaaaaaaggatatagagtcaaaggaaagtaaaaagcctgaggcagaaagaa aagagagtacacaaaacagggctgagagatttaaagaatcattaattacttcagagaatgac aatgttgttcagtatacaaatgaagacggtgaaatacgaaccatcatatgggtaaagggaatt aacggacctgttatgggagaaggtggagactttcataaagagattacacaaggcggtaagaa tacttatgtaatgtatactgtaccatattcaagcggagacggatggtatgatgttaataaaacaag aagcggtggaaatatcgatatagataaaaatctttgctttgcagcggtatcatcaaatatgcttca ttggtggtttgatcaaaatattgaatatgttgacagatatatagaaaaaaacggagatataattcg tgccaacagaaaactttcagatttaaaaacttcctttgaaaatccggaaaacagtaaaatatttg agctgtataaagtattgtacggatataatgagagagggttttattcggatcttttgatggatttatttat taacggatatactccaaacccaaagggtggaacaaatatagaaagtgatgatcttatcccaa ataataacggcggattcttctatgatgttttcaaggggaaaaacttacagaccgtacttatggtg gaaactatgaatcactaagtaatcttcttaaagaaatcttgggtaatggcggcatagtaggacttt ctcataaagtgttttcaaagagtaatcatatagttactttgtggggtgccgaatatgacttgaacgg gaagttgaaggctgtatatataagcgattctgacgatcaggatgaaagtaatgtaggcatgaa acgttttgaaatccgaaatgtaggaggaaaagcaaaaatatctaccaacgaaacagataagt ctgcgggtgctgaattaggatatcttcatatcctatatcagggaaccggacaatgggaaaattat tttaaataa |
| Encodes SEQ ID NO: 9 | |
| SEQ ID NO: 21 | atgggtgtggacggctggaaacaggagaacaacagctggaagtactataaagaagataag gcgctgaaagcgtggcagcaaatcaacaacaactggtatttctttaacgaggacggtagcctg aaaaccggctggtacatggacgcggcgaacaactggtatttcctggatagcagcaagaccg cgaacgagggtgttctgctgagcggctggcagtggatcgacggttactgctactatttcgaagg |
| Encodes SEQ ID | |
| NO: 10 | cattgataacaacacctttggtaaaatgtacagcaacaccctggttagcggctataaggttgac gcgctgggtcgttggatcaacgagagcggcgcggaatactatgaggcgggtaaaggcatta gcaccggtaacgcgagccaaagcagcagcaacaccgcgaagaaaaccagcgtggcggg tagcagcgaaaagagcggcaaaagcggtggcagcagcaagggtggcagcagcggtaag agcggtggcagcagcaagggtggcagcagcgggaaaagcggtggcagcagcttcctgaa ccagggcgagaagcgtgaggaaaaccgtatcctgggtgaaagcaagggcaaagcggag aacagcagcagcaaacgtagcgaagacctggttggtagccgtcaagacctgagcagcgat aagaacaacagcctgatcgcggataacaagaccggtaacagcgaaaaagcgggcagcat tgagaagaacaccaacaaagaatataccaccaagagcaacgagagcccggtgaaaccg gcgaccagcagcaacgttgaaatcccgaaggaagaggagaagaaggacattgaaagca aagagagcaagaaaccggaagcggagcgtaaggagagcacccagaaccgtgcggaac gtttcaaagagagcctgatcaccagcgaaaacgacaacgtggttcaatataccaacgaagat ggcgagatccgtaccatcatttgggtgaagggtattaacggcccggttatgggcgagggtggc gattttcacaaggaaattacccagggtggcaaaaacacctacgtgatgtataccgttccgtaca gcagcggtgacggctggtatgatgtgaacaaaacccgtagcggtggcaacatcgacattgat aagaacctgtgcttcgcggctgtgagcagcaacatgctgcactggtggtttgaccaaaacatc gagtacgttgatcgttatattgagaagaacggtgacatcattcgtgcgaaccgtaagctgagcg atctgaaaaccagcttcgaaaacccggagaacagcaagatctttgagctgtacaaagtgctgt acggttataacgaacgtggcttctatagcgacctgctgatggacctgttcattaacggttacacc ccgaacccgaagggtggcaccaacatcgagagcgacgatctgattccgaacaacaacggt ggcttcttttacgacgttttcaaaggcaagaaactgaccgatcgtacctacggtggcaactatga gagcctgagcaacctgctgaaggaaatcctgggtaacggtggcattgtgggcctgagccaca aggtttttagcaaaagcaaccacatcgtgaccctgtggggtgcggagtacgacctgaacggc aagctgaaagcggtttatattagcgacagcgacgatcaagatgagagcaacgtgggtatgaa acgtttcgaaatccgtaacgttggtggcaaggcgaaaattagcaccaacgagaccgataaga gcgcgggtgcggaactgggctacctgcacatcctgtatcagggtaccggccaatgggaaaa ctactttaagctcgagcaccaccaccaccaccactga |
| SEQ ID NO: 22 | atgcaccaccaccaccaccaccaccaccaccacggtgtggacggctggaaacaggagaa caacagctggaagtactataaagaagataaggcgctgaaagcgtggcagcaaatcaacaa caactggtatttctttaacgaggacggtagcctgaaaaccggctggtacatggacgcggcgaa caactggtatttcctggatagcagcaagaccgcgaacgagggtgttctgctgagcggctggca gtggatcgacggttactgctactatttcgaaggcattgataacaacacctttggtaaaatgtaca gcaacaccctggttagcggctataaggttgacgcgctgggtcgttggatcaacgagagcggc gcggaatactatgaggcgggtaaaggcattagcaccggtaacgcgagccaaagcagcagc aacaccgcgaagaaaaccagcgtggcgggtagcagcgaaaagagcggcaaaagcggtg gcagcagcaagggtggcagcagcggtaagagcggtggcagcagcaagggtggcagcag cgggaaaagcggtggcagcagcttcctgaaccagggcgagaagcgtgaggaaaccgtat cctgggtgaaagcaagggcaaagcggagaacagcagcagcaaacgtagcgaagacctg gttggtagccgtcaagacctgagcagcgataagaacaacagcctgatcgcggataacaaga ccggtaacagcgaaaaagcgggcagcattgagaagaacaccaacaaagaatataccacc aagagcaacgagagcccggtgaaaccggcgaccagcagcaacgttgaaatcccgaagg aagaggagaagaaggacattgaaagcaaagagagcaagaaaccggaagcggagcgta aggagagcacccagaaccgtgcggaacgtttcaaagagagcctgatcaccagcgaaaac gacaacgtggttcaatataccaacgaagatggcgagatccgtaccatcatttgggtgaagggt attaacggcccggttatgggcgagggtggcgattttcacaaggaaattacccagggtggcaa aaacacctacgtgatgtataccgttccgtacagcagcggtgacggctggtatgatgtgaacaa aacccgtagcggtggcaacatcgacattgataagaacctgtgcttcgcggctgtgagcagca acatgctgcactggtggtttgaccaaaacatcgagtacgttgatcgttatattgagaagaacggt gacatcattcgtgcgaaccgtaagctgagcgatctgaaaaccagcttcgaaaacccggaga acagcaagatctttgagctgtacaaagtgctgtacggttataacgaacgtggcttctatagcgac ctgctgatggacctgttcattaacggttacaccccgaacccgaagggtggcaccaacatcgag agcgacgatctgattccgaacaacaacggtggcttcttttacgacgttttcaaaggcaagaaac tgaccgatcgtacctacggtggcaactatgagagcctgagcaacctgctgaaggaaatcctg ggtaacggtggcattgtgggcctgagccacaaggtttttagcaaaagcaaccacatcgtgacc ctgtggggtgcggagtacgacctgaacggcaagctgaaagcggtttatattagcgacagcga |
| Encodes SEQ ID NO: 11 | |
| | cgatcaagatgagagcaacgtgggtatgaaacgtttcgaaatccgtaacgttggtggcaagg cgaaaattagcaccaacgagaccgataagagcgcgggtgcggaactgggctacctgcaca tcctgtatcagggtaccggccaatggaaaactactttaagctcgagtga |
| SEQ ID NO: 23 | MLMSFNAYAGADGWKQENNKWKYYKEDKALKAWQQINNNWYFF NEDGSLKTGWYMDASNNWYFLDSSKTANEGVLLSGWQWIDGYC YYFEGTDNTTFGKMYSNTDVSGYRVDALGRWINESGTEYYEAGK GISTGNASQSSSNTAKKQGNAGGLGKSSGSGGLGKGESSGKGGS SGKSRGLGRGGSLGKDGGSDSLGKGESYDKSAGSNKGENTGRS DDLSNLNQGEKREESRILRESENGGSFSRSEGSISQRKELSQDKE DTQKSNESPVKPATSSNVEIPKEEEKKDIEPKESKKLEAKRKESTQ DRDEKIKESLITPENDNVVQYTTEDGEIRTIIWAKGINGPLMGEGGD FHKEIIQGGKDTYITYTVPYSSGDGWYDVNKTTSGGNIDIDKNLCFA AVSSNMLHWWFDQNIEYVDRYIEKNGDIIRANRKLSDLKTSFESQE KSEIFELYKVLYGYNERGFYSDLLMDLFINGYTPKITGATNIESDDLIP SNNGGFFHDVFKGEKLTERTYGGNYQSLSDLLKEIFGNGGIVGVSH KVFRKSNHIVTLWGAEYDLNGKLKAVYVSDSDDQDERSVGMKRYE IRNVGGRAKLSTNETDKSAGAEVGYLHILYLGTNQWNDYFR |
| Enzyme according to accession no. EJP19400.1 | |
| SEQ ID NO: 24 | MKNIQKVCMITCMVSVFSFFCGSLGSYAMDAAGTDRVGQVTEYID EGGEKRTIYWADHITPPKMGGGDSEFQKHEYEQGGTRYVEYIAPY TAGNGWYDVNKTPDQTQDANLCFSAAAANTLHWWLDQNTTYIDR YLAAYPEAPKAEEIRELKGQTPGQHDSKIYARFVEQFANRTTGNWP DILQDQFINGYPPKENGGTNDPEYDGKKLMETGPDKRGGFFYPVF GPDILTQRRFYDFTGNYQSISADMKQFFEQGQIVTLTYKLWESAHV VTVWGAEYDSAGNLSGVYFSDSDDMDEYGMHRYRVVNKKGQAY VTTDMSDDGGGSPITCLTTLSTGTRYWEKVLTPTELELVWGDTQFV YNGSPQIPTLTAGNIADGDDAWLSVKGEGVEVGTYSASAMLSGND AGKYSLPAEDAGVSFVITQSGTVFEGGVKAYLGTKETTDVQYGDTI TIKVSPRATGNAPDSSEPPSVSVKPTNGQVTFFAGGQKLNEAVNTD ENGVYTLTYDTKKGALKAGANQLTVKFHDDKNMADHEEKLTIFVKA AEHIPGGEEPPAQEGNGEKAPVYGENGEKADRADRAVKTGDDTS GLIWMFISAGAAAVVYGLYVRNRKKA |
| Enzyme according to accession no. WP_162858205.1 | |
| SEQ ID NO: 25 | MQKFNKILAPAFLGLSMSLLFAVSSYASVEGWKVENGTWKYYKEYK ALKSWQKLGNNWYFFNEDGSLKTGWLKDGNDNWYFLDLNSGSN QGVLLSGWQWIDGYCYYFETSDSGTLGRMYADKTVDGYKLNPMG RWVDNNGKELYDATRGISTNKQNLSSDKDLAKKASIAAKRSGSAG GVSRGSGGASSGGGSSRSTGSSYSYSSGNSNFSNSASKHSSDSD NTGSSYSYDDGSLSNSNSNSTKDNLIQSNNNSVSTDNSLNSAGGS SSANVGSSSNSGLSSSLGSVTKPSTGLVSQNQGSLSSDSNNKTNA DSKQNTVPDKSNNKKFEDTNTENPKTGNEHSTPPSASENSAGSNT KGSLTPAAEHGDNTDPTEDTTATSSNAAPKPENKEIPNATEDSKKQ ENENKAKEQEENKADKVKDLLTTEKNNNVVQYTDEDGKIRTIIWAK GISAPIMGEGGDFRKEVTHAGSDTYIDYKAPFTFGNSWYDSNKSR AGGNTDIDKNLCFGATSANMLHWWFEQNSSYIDSYIAKEGDITRSG RSLSELRNSFNNQQDSGIFELFKVLFGYNDKGFYSDLLMDLFINGY TPKISGATNIENEDMTPDNRGGFFYNAFKEVKLTERTYGGSYEDLS EKLKDILGNEGIIGISHKTFGNSSHIVTLWGAEYDLNGKLSAVYVSDS DDQNEEADETDLGMKRYEVRNVGGIAKLSTNQSNKSAGSAIGYLH VLYLGSEQWQSYINQ |
| Enzyme according to accession no. RKW40033.1 | |
| SEQ ID NO: 26 | MGWDQDSQVKDGLAEFRAYHTAEKHSQKLVRNHTQKYIDKTIGAR KNGGHPFSVFSRSFMLSIPLALTLPFFSLTAQAEGKWVQAEGQWQ YQEEGQNQKGWKEISGSWYYFDPLTGKMQDGWILADGKWYFLKT DTANWGKMAQGWTWVDGYCYFLKDNGTMATEEKTSDGYSISAN GQWVQDGKPVHEEGKGVSSRRSAQVEKEIKAGVAEGQQINANGE AQNTGIKGIAGSGGSGGSGGGGGFSGGSGSGGGSSFGGSSSSG GFGSGSGSSFASASGSTSSASGSSASSVGGGFHFGAGNSSGTVE |
| Enzyme according to accession no. WP_007157024.1 | |
| | SDSRQRDLFGDSKYDSVSNTEDNTGFSPEATEKENTFSEEELARR AEKQRKQAEKELAKRKKLLEKIENGQNNNTVEYETEEGEKRTVLFV KGVKAPKLGENGDFRKTEDHGYIDYKAPYEEGQGYFDVNKAPFGT NKEIDRNLCFAAAASNTLHWYLQQNKKEIEDYIKNNGDVIRTVGANT YSLKDMLNQDVEQQGSLIYQYFKEMYGNNETGYYTVPLMDLFLNG YTPKEDRKTNIENKDLQPDARGGFLYGIIGTKPQTGMHLVNSLSDL GNSLQHYLSNNFVVCLSYTTFSYNHVVTLWGAEYDESGLLRAVYV TDSDDQDETGVETDVAMKRYVVKGNGNLSYLSNAISEEAKGARIN SLQYLRFGGEEDLED |
| SEQ ID NO: 27 | GQQMETGDQRFGDLVFRQLAPNVWQHTSYLDMPGFGAVASNGLI VRDGGRVLVVDTAWTDDQTAQILNWIKQEINLPVALAVVTHAHQDK MGGMDALHAAGIATYANALSNQLAPQEGMVAAQHSLTFAANGWVE PATAPNFGPLKVFYPGPGHTSDNITVGIDGTDIAFGGCLIKDSKAKS LGNLGDADTEHYAASARAFGAAFPKASMIVMSHSAPDSRAAITHTA RMADKLR |
| Amino acid sequence of NDM-1 protein | |
| SEQ ID NO: 28 | MKYLLPTAAAGLLLLAAQPAMAMAAHHHHHHSSGVDLGTENLYFQ GQQMETGDQRFGDLVFRQLAPNVWQHTSYLDMPGFGAVASNGLI VRDGGRVLVVDTAWTDDQTAQILNWIKQEINLPVALAVVTHAHQDK MGGMDALHAAGIATYANALSNQLAPQEGMVAAQHSLTFAANGWVE PATAPNFGPLKVFYPGPGHTSDNITVGIDGTDIAFGGCLIKDSKAKS LGNLGDADTEHYAASARAFGAAFPKASMIVMSHSAPDSRAAITHTA RMADKLR |
| Recombinantly expressed NDM-1 construct including a PelB leader sequence, a His-tag and a TEV protease recognition site | |
| SEQ ID NO: 29 | HHHHHHSSGVDLGTENLYFQGQQMETGDQRFGDLVFRQLAPNV WQHTSYLDMPGFGAVASNGLIVRDGGRVLVVDTAWTDDQTAQILN WIKQEINLPVALAVVTHAHQDKMGGMDALHAAGIATYANALSNQLA PQEGMVAAQHSLTFAANGWVEPATAPNFGPLKVFYPGPGHTSDNI TVGIDGTDIAFGGCLIKDSKAKSLGNLGDADTEHYAASARAFGAAF PKASMIVMSHSAPDSRAAITHTARMADKLR |
| Corresponds to SEQ ID NO: 28 but with the PelB leader sequence cleaved off | |
| SEQ ID NO: 30 | MELPNIMHPVAKLSTALAAALMLSGCMPGEIRPTIGQQMETGDQRF GDLVFRQLAPNVWQHTSYLDMPGFGAVASNGLIVRDGGRVLVVDT AWTDDQTAQILNWIKQEINLPVALAVVTHAHQDKMGGMDALHAAGI ATYANALSNQLAPQEGMVAAQHSLTFAANGWVEPATAPNFGPLKV FYPGPGHTSDNITVGIDGTDIAFGGCLIKDSKAKSLGNLGDADTEHY AASARAFGAAFPKASMIVMSHSAPDSRAAITHTARMADKLR |
| NDM-1 from Klebsiella NCBI ID: NG_049326.1 | |
| SEQ ID NO: 31 | MAEVISGSAVTLNTNMTKNVQNGRAYIDLYDVKNGKIDPLQLITLNS PDLKAQYVIRQGGNYFTQPSELTTVGAASINYTVLKTDGSPHTKPD GQVDIINVSLTIYNSSALRDKIDEVKKKAEDPKWDEGSRDKVLISLD DIKTDIDNNPKTQSDIANKITEVTNLEKILVPRIPEFSTIPGSAATLNTS ITKNIQNGNAYIDLYDVKLGKIDPLQLIVLEQGFTAKYVFRQGTKYYG DVSQLQSTGRASLTYNIFGEDGLPHVKTDGQIDIVSVALTIYDSTTLR |
| The sequence of a first immunogenic | |
| fusion protein comprising the N-terminals or Rib and AlpC for use in a GBS vaccine | DKIEEVRTNANDPKWTEESRTEVLTGLDTIKTDIDNNPKTQTDIDSKI VEVNELEKLLVLS |
| SEQ ID NO: 32 | MAEVISGSAATLNSALVKNVSGGKAYIDIYDVKNGKIDPLNLIVLTPS NYSANYYIKQGGRIFTSVNQLQTPGTATITYNILDENGNPYTKSDGQ IDIVSLVTTVYDTTELRNNINKVIENANDPKWSDDSRKDVLSKIEVIK NDIDNNPKTQSDIDNKIVEVNELEKLLVLPEFSTIPGSAATLNTSITKN IQNGNAYIDLYDVKNGLIDPQNLIVLNPSSYSANYYIKQGAKYYSNPS EITTTGSATITFNILDETGNPHKKADGQIDIVSVNLTIYDSTALRNRIDE VINNANDPKWSDGSRDEVLTGLEKIKKDIDNNPKTQIDIDNKINEVN EIEKLLVVSL |
| The sequence of a second immunogenic fusion protein comprising the N-terminals or Alp1 and Alp2 for use in a vaccine | |
| SEQ ID NO: 33 | MAEVISGSAVTLNTNMTKNVQNGRAYIDLYDVKNGKIDPLQLITLNS PDLKAQYVIRQGGNYFTQPSELTTVGAASINYTVLKTDGSPHTKPD GQVDIINVSLTIYNSSALRDKIDEVKKKAEDPKWDEGSRDKVLISLD DIKTDIDNNPKTQSDIANKITEVTNLEKILVPRIP |
| The amino acid sequence of the N-terminal of Rib | |
| SEQ ID NO: 34 | STIPGSAATLNTSITKNIQNGNAYIDLYDVKLGKIDPLQLIVLEQGFTA KYVFRQGTKYYGDVSQLQSTGRASLTYNIFGEDGLPHVKTDGQIDI VSVALTIYDSTTLRDKIEEVRTNANDPKWTEESRTEVLTGLDTIKTDI DNNPKTQTDIDSKIVEVNELEKLLVLS |
| The amino acid sequence of the N-terminal of AlpC | |
| SEQ ID NO: 35 | MAEVISGSAATLNSALVKNVSGGKAYIDIYDVKNGKIDPLNLIVLTPS NYSANYYIKQGGRIFTSVNQLQTPGTATITYNILDENGNPYTKSDGQ IDIVSLVTTVYDTTELRNNINKVIENANDPKWSDDSRKDVLSKIEVIK NDIDNNPKTQSDIDNKIVEVNELEKLLVLP |
| The amino acid sequence of the N-terminal of Alp1 | |
| SEQ ID NO: 36 | STIPGSAATLNTSITKNIQNGNAYIDLYDVKNGLIDPQNLIVLNPSSYS ANYYIKQGAKYYSNPSEITTTGSATITFNILDETGNPHKKADGQIDIV SVNLTIYDSTALRNRIDEVINNANDPKWSDGSRDEVLTGLEKIKKDID NNPKTQIDIDNKINEVNEIEKLLVVSL |
| The amino acid sequence of the N-terminal of Alp 2 and Alp3 | |

The technology proposed herein will be further described in the following examples, which do not limit the scope of the invention described in the claims.

### Example 1 - Cleavage, inactivation or removal of IgM in infant and cord blood samples required to separate Rib/CPSIII cases and controls in OPkA

An opsonophagocytic killing assay (OPkA) is designed to measure the functionality of antibodies present in blood serum samples and to assess protective immunity.
In this example, blood serum samples from infants who had contracted GBS infection naturally ("Cases") and who did not ("Controls") were obtained and analyzed in an OPkA for efficacy in killing GBS bacteria of the Rib/CPSIII strains. The purpose of the example was to evaluate if there was a significant difference in OPkA titer levels in the respective blood samples which could separate the Cases from the Controls. If such a difference was found, then a GBS vaccine candidate which, when administered to infants or pregnant females, provided the same or higher OPkA titer levels as the Controls, could be considered to provide protection against GBS bacteria expressing Rib/CPSIII. In other words, the example sought to define a surrogate endpoint and surrogate efficacy test for a GBS vaccine candidate since the true test, i.e. immunizing infants using the vaccine candidate and then exposing the infants to GBS bacteria to determine whether the vaccine candidate was effective or not as determined by the clinical endpoint of disease or no disease, was not possible.

This example assayed blood samples that had been obtained by healthcare institutions, e.g. hospitals, and then stored in biobanks from which samples were retrieved for performing the example. The blood samples were randomly selected cases and controls from a South African seroepidemiological study.

The blood samples, i.e. serum samples, were heat-treated to inactivate human complement molecules present in the sample. The samples were then treated with 200 nM (5ug/mL) NDM-1 enzyme for 30 minutes to remove any beta-lactam antibiotic that could be present in the sample.

The samples were then either subjected to treatment by IgM cleavage (IgM cleaved) or left untreated (plain) before the OPkA. The IgM cleaved samples (both cases and controls) were treated with the enzyme IgMBRAZOR^{™} (Genovis, product Number M0-IM1-010, molecular weight of 38 kDa) at 37°C for 16-18 h. IgMBRAZOR^{™} is an IgM-specific enzyme that digests human IgM at one specific site below the CH2 domain in the heavy chain, generating F(ab')2 and Fc fragments, and it does not digest IgG or IgA. 0.8 For cleaving IgM, 0.8 units/µL IgMBRAZOR^{™} was added to the samples held at 37°C overnight, before the samples were serially diluted using opsonization buffer as described below. Other suitable enzymes for cleaving human IgM are defined above.

The OPkA was performed as follows. A GBS strain expressing Rib/CPSIII was cultured to the log phase. The samples were serially diluted using opsonization buffer (buffer details: 0.95% Hanks' balanced salt solution containing Ca²⁺ and Mg²⁺ ions, 0.1% gelatin, and 5% heat inactivated Fetal Bovine Serum) and incubated with the target bacteria for 30 minutes at 37°C. The samples were then incubated with a mixture of baby rabbit complement (12.5%) and differentiated HL-60 cells (400 000 cell/well) for 30 minutes at 37°C. The ratio effector cell/target is 200/1. From the assay reaction, 5 µl were plated on THY agar plates and incubated at 37°C in a 5% CO₂ atmosphere for 16-18 hours. The CFU was counted and the OPkA titer was reported as the reciprocal of the dilution factor required to achieve 50% bacterial killing compared to the control well, which contains everything but no serum.

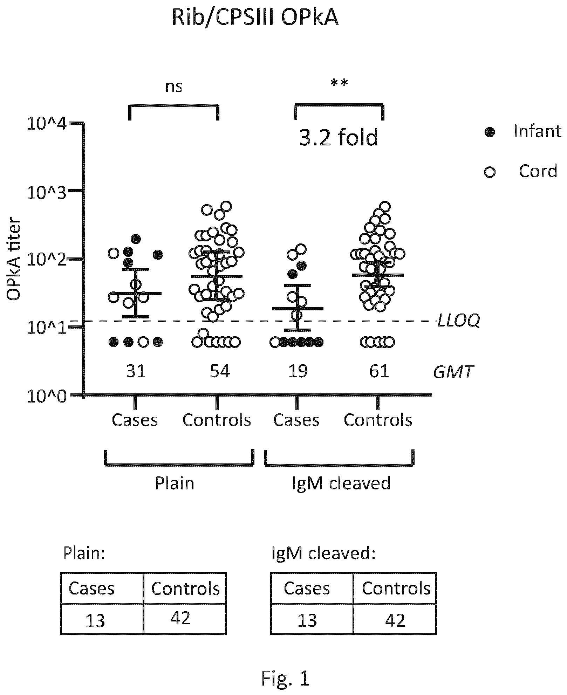

Fig. 1, left, shows the OPkA titers for untreated (plain) samples. For the group Cases, i.e. infants having contracted or been diagnosed with the disease at or in connection with birth, blood samples were either from the infant, i.e. marked "Infant" or from the umbilical cord, i.e. marked "Cord", the former having precedence. The group Controls contained only blood samples from the umbilical cord since these infants were generally born healthy and thus only the routine cord blood samples had been obtained from them.

As seen, there is no significant difference in OPkA titer between Cases and Controls with Cases having a geometric mean titer (GMT) of 31 and Controls having a mean OPkA titer of 54. This was surprising since about 20 % of all women are vaginal carriers of GBS while only about 1 % of the infants that are colonized by the GBS at birth are afflicted by serious infection. Accordingly, it must be expected that not only the "cases" group (who evidently was exposed to the GBS bacteria and contracted the disease), but also the "Controls" group had been exposed to the GBS bacteria but did not contract the disease. The infants in the Control group must therefore have had protective immunity against GBS bacteria. However, the evident difference in immunity between the Control group and the Cases group was as shown not reflected in the OPkA titer between Cases and Controls.

At this point, the present inventors hypothesized that some of the OPkA titer results for the Cases group could be due to the presence of antibodies in the sample that, while being detected by the OPkA, i.e. were effective in killing GBS bacteria in the assay, were not in fact effective in providing protective immunity to GBS-bacteria at the time of infection, i.e. in the infant. IgM and IgG are both antibody subclasses that are used by the human immune system to combat infectious agents. As such, they could both influence the results of an opsonization assays. It however appeared unlikely that IgM from the mother could be involved since IgM is not placentally transferred to the infant.

The inventors nevertheless treated the samples with an enzyme cleaving IgM to see if any improvement in resolution could be seen.

Fig. 1, right shows the OPkA titers for treated (IgM cleaved) samples. Surprisingly, this reduced the mean OPkA titer of the Cases group to 19 and slightly increased the OPkA titer of the Controls group to 61 such that a statistically significant (p<0.01) 3.2-fold difference between the Cases group and the Controls group. In other words, after removing IgM from the samples it was possible to define a mean OPkA titer for Controls at 61 which differed significantly from the mean OPkA titer of the Cases at 19. Similar results were obtained if the Cases group was divided based on early onset of disease (EOD) or late onset disease (LOD). Here the GMT of plain samples was 33 for EOD and 30 for LOD, which decreased to 18 and 19 for treated samples.

Without wishing to be bound by theory it thus appeared that IgM in the samples contributed to a protective effect in the assay but not in the infant at the time of exposure to the GBS bacteria. This IgM could possibly be polyreactive innate IgM produced already during fetal life, IgM produced in the infant in response to the exposure or infection but not reaching protective levels until the respective sample was collected, or IgM present in maternal blood contaminating the samples.

Accordingly, the inactivation of IgM in the samples allows for separating Cases from Controls and is thus useful when determining a surrogate endpoint for a vaccine against GBS. The step of removing, inactivating or cleaving IgM may be applied to any infant samples and cord blood samples whether the samples relate to clinical trials or seroepidemiological studies.

To establish a surrogate endpoint or surrogate efficacy marker (SEM) for invasive GBS disease (iGBSd) based on vaccine induced IgG-driven OPkA measurements, IgM present in infant blood samples due e.g. to delayed blood collection in relation to disease onset or maternal contamination, needs to be removed as it prevents accurate analysis of functional GBS specific IgG responses transferred from mother to infants, e.g. following vaccination of the mother using a GBS vaccine.

In other words, inactivation of IgM allows a correct assessment of the GBS IgG-mediated OPkA titers required for protection against GBS disease in the infant, and also the levels of functional post-vaccination IgG transferred from mother to infant after the mother has been vaccinated, such as by administering a vaccine comprising the fusion proteins of SEQ ID NO: 31 and 32 to the mother. In addition, inactivation of IgM removes any IgM produced by the infant between infection and sampling, e.g. due to any significant delay between infection and sampling, thereby allowing the assessment of the IgG-driven OPkA activity in the sample before the infection occurred.

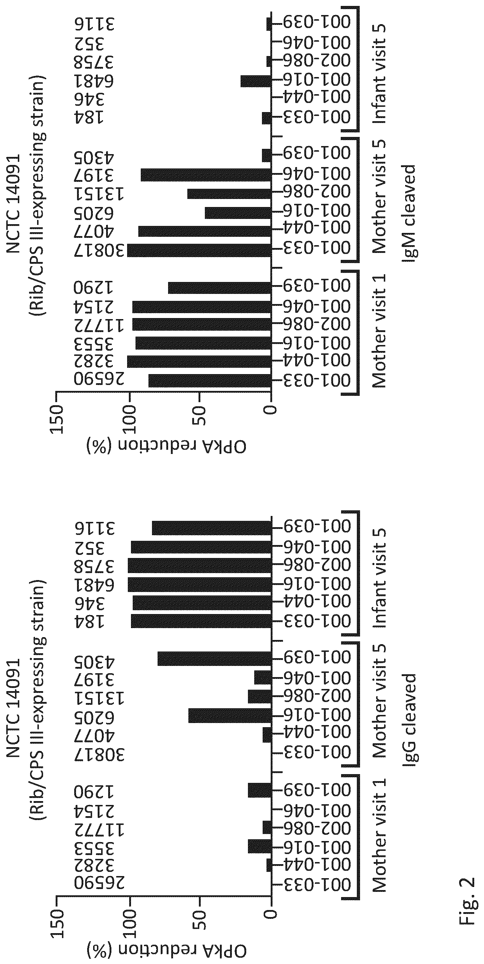

To further ascertain the protective effect of administering a vaccine comprising the fusion proteins of SEQ ID NO: 31 and 32, blood samples from mothers pre-vaccination (visit 1) and post-vaccination (visit 5), as well as corresponding cord samples (Visit 5) were subjected to IgM or IgG cleavage and tested for OPk activity in an OPkA, see Fig. 2.

Regardless of maternal IgM levels pre- (Visit 1) and post-vaccination (Visit 5 - delivery visit), OPk activity exclusively driven by IgG is observed in cord samples for Rib/CPS III. Additionally, a strong reduction (58-96%) in cord OPkA titer was observed for the RibN/CPS III strain after inhibition of these cord samples with soluble RibN protein. Together, these data demonstrate both the high relative contribution, and the functionality of vaccine-induced AlpN-specific IgG transferred from the mother to the infant.

A surrogate endpoint may be obtained from the data shown in Fig. 1 by applying statistical modelling to e.g. establish a 70%, 80%, or 90% threshold value for protection against disease.

In other words, a threshold value, i.e. a surrogate endpoint, may be obtained by statistical calculations on the data in Fig. 1, e.g. assuming a suitable distribution of the OPkA titers, to determine an OPkA titer, or a bracket or interval of OPkA titers, at which the probability of the subject being protected against disease being e.g. 70%, 80%, or 90%.

Alternatively, the surrogate endpoint may be obtained by fitting a statistical model to the data shown in Fig. 1 allowing estimation of protection against disease

Once the surrogate endpoint has been obtained as discussed above, the surrogate efficacy test method may proceed to:
vaccinate at least one human, preferably a plurality of humans, using a vaccine against GBS,
removing, inactivating and/or cleaving IgM in a sample from the at least one human and obtaining a measurement of immunological activity, e.g. an OPkA titer, and
comparing the measurement with the surrogate endpoint.
The surrogate efficacy test method thus allows for ascertaining whether the vaccine against GBS can induce protection against GBS disease.

The technology proposed herein is further illustrated by the following points:
1. A method of treating a sample, wherein the sample comprises blood, serum, and/or plasma from a human infant or from a human umbilical cord, wherein the method comprises:
   (i) removing, inactivating, and/or cleaving human IgM in the sample.
2. The method of point 1, wherein step (i) is performed by treating the sample with an enzyme to cleave human IgM in the sample.
3. The method of point 2, wherein the enzyme is from *Lachnoanaerobaculum sp. ICM7, Mediterraneibacter massiliensis, Lachnospiraceae bacterium,* or *Oribacterium Sinus.*
4. The method of point 2, wherein the gene encoding the enzyme is selected from the group consisting of accession IDs: EJP19400.1, WP_162858205.1, RKW40033.1, and WP_007157024.1.
5. The method of point 2, wherein the enzyme comprises a polypeptide having at least 80% sequence identity to SEQ ID NO: 1, SEQ ID NO: 2, SEQ ID NO: 8 or SEQ ID NO: 9.
6. The method of point 2, wherein the enzyme comprises a polypeptide having at least 90% sequence identity to SEQ ID NO: 1, SEQ ID NO: 2, SEQ ID NO: 8 or SEQ ID NO: 9.
7. The method of points 5 or 6, wherein the polypeptide comprises an additional methionine at the N terminus; and/or a His tag at the C terminus or at the N-terminus.
8. The method of any of points 5-7, wherein the polypeptide comprises or consists of a sequence selected from the group consisting of SEQ ID NOs: 3, 4, 5, 6, 7, 10, and 11.
9. The method of point 8, wherein the polypeptide comprises or consists of SEQ ID NO: 7.
10. The method of any of points 1-9, further comprising:
   (ii) treating the sample with a further enzyme to inactivate an antimicrobial substance in the sample.
11. The method of point 10, wherein the further enzyme is a beta-lactamase.
12. The method of point 11, wherein the beta-lactamase is a carbapenemase.
13. The method of point 12, wherein the carbapenemase is a class B carbapenemase.
14. The method of point 13, wherein the class B carbapenemase comprises a New Delhi metallo-beta-lactamase 1 (NDM-1) polypeptide.
15. The method of point 14, wherein the New Delhi metallo-beta-lactamase 1 (NDM-1) polypeptide comprises a further polypeptide having at least 95% sequence identity to SEQ ID NO: 27 or 28.
16. The method of point 15, wherein the New Delhi metallo-beta-lactamase 1 (NDM-1) polypeptide comprises a further polypeptide having at least 95% sequence identity to SEQ ID NO: 28.
17. A method of obtaining a measurement of immunological activity of a sample, comprising:
   (i) removing, inactivating, and/or cleaving human IgM in the sample,
   (ii) obtaining a measurement of immunological activity of the sample against a microbe.
18. The method of point 17, wherein the microbe is a GBS bacterium.
19. The method of point 18, wherein the GBS bacterium expresses the surface proteins Rib or Alp2.
20. The method of point 19, wherein the GBS bacterium expresses the surface protein Rib.
21. The method of point 19, wherein the GBS bacterium expresses the surface protein Alp2.
22. The method of point 18, wherein the GBS bacterium belongs to the Rib/CPSIII or Alp2/CPSV GBS serotype.
23. The method of point 22, wherein the GBS bacterium belongs to the Rib/CPSIII GBS serotype.
24. The method of point 22, wherein the GBS bacterium belongs to the Alp2/CPSV GBS serotype.
25. The method of any of the points 17-24, wherein the measurement of immunological activity is obtained using an Opsonophagocytic killing Assay (OPkA).
26. The method of any of points 17-25, wherein step (i) is performed by treating the sample with an enzyme to cleave human IgM in the sample.
27. The method of point 26, wherein the enzyme is from *Lachnoanaerobaculum sp. ICM7, Mediterraneibacter massiliensis, Lachnospiraceae bacterium,* or *Oribacterium Sinus.*
28. The method of point 26, wherein the gene encoding the enzyme is selected from the group consisting of accession IDs: EJP19400.1, WP_162858205.1, RKW40033.1, and WP_007157024.1.
29. The method of point 26, wherein the enzyme comprises or consists of a polypeptide having at least 80% sequence identity to SEQ ID NO: 1, SEQ ID NO: 2, SEQ ID NO: 8 or SEQ ID NO: 9.
30. The method of point 26, wherein the enzyme comprises or consists of a polypeptide having at least 90% sequence identity to SEQ ID NO: 1, SEQ ID NO: 2, SEQ ID NO: 8 or SEQ ID NO: 9.
31. The method of points 29 or 30, wherein the polypeptide comprises an additional methionine at the N terminus; and/or a His tag at the C terminus or at the N-terminus.
32. The method of any of points 29-31, wherein the polypeptide comprises or consists of a sequence selected from the group consisting of SEQ ID NOs: 3, 4, 5, 6, 7, 10, and 11.
33. The method of any of points 32, wherein the polypeptide comprises or consists of SEQ ID NO: 7.
34. The method of any of points 17-33, wherein (i) further comprises treating the sample with a further enzyme to inactivate an antimicrobial substance in the sample.
35. The method of point 34, wherein the further enzyme is a beta-lactamase.
36. The method of point 35, wherein the beta-lactamase is a carbapenemase.
37. The method of point 36, wherein the carbapenemase is a class B carbapenemase.
38. The method of point 37, wherein the class B carbapenemase comprises a New Delhi metallo-beta-lactamase 1 (NDM-1) polypeptide.
39. The method of point 38, wherein the New Delhi metallo-beta-lactamase 1 (NDM-1) polypeptide comprises a further polypeptide having at least 95% sequence identity to SEQ ID NO: 27 or 28.
40. The method of point 39, wherein the New Delhi metallo-beta-lactamase 1 (NDM-1) polypeptide comprises a further polypeptide having at least 95% sequence identity to SEQ ID NO: 28.
41. A method of obtaining a surrogate endpoint, comprising:
   (i) obtaining at least one sample associated with at least one of:
      (a) a first group of infants not afflicted by a disease or an infection after being exposed to a microbe, or
      (b) a second group of infants afflicted by a disease or an infection after being exposed to a microbe, and
   (ii) obtaining a measurement of immunological activity of the at least one sample, comprising:
      (a) removing, inactivating, and/or cleaving human IgM in the at least one sample,
      (b) obtaining a measurement of immunological activity of the at least one sample against a microbe, and
   (iii) determining the surrogate endpoint based on a plurality of measurements.
42. The method of point 41, wherein the microbe is a GBS bacterium.
43. The method of point 42, wherein the GBS bacterium expresses the surface proteins Rib or Alp2.
44. The method of point 43, wherein the GBS bacterium expresses the surface protein Rib.
45. The method of point 44, wherein the GBS bacterium expresses the surface protein Alp2.
46. The method of point 41, wherein the GBS bacterium belongs to the Rib/CPSIII or Alp2/CPSV GBS serotype.
47. The method of point 46, wherein the GBS bacterium belongs to the Rib/CPSIII GBS serotype.
48. The method of point 46, wherein the GBS bacterium belongs to the Alp2/CPSV GBS serotype.
49. The method of any of the points 41-48, wherein the measurement of immunological activity is obtained using an Opsonophagocytic killing Assay (OPkA).
50. The method of any of points 41-49, wherein step (ii)(a) is performed by treating the at least one sample with an enzyme to cleave human IgM in the sample.
51. The method of point 50, wherein the enzyme is from *Lachnoanaerobaculum sp. ICM7, Mediterraneibacter massiliensis, Lachnospiraceae bacterium,* or *Oribacterium Sinus.*
52. The method of point 50, wherein the gene encoding the enzyme is selected from the group consisting of accession IDs: EJP19400.1, WP_162858205.1, RKW40033.1, and WP_007157024.1.
53. The method of point 50, wherein the enzyme comprises or consists of a polypeptide having at least 80% sequence identity to SEQ ID NO: 1, SEQ ID NO: 2, SEQ ID NO: 8 or SEQ ID NO: 9.
54. The method of point 50, wherein the enzyme comprises or consists of a polypeptide having at least 90% sequence identity to SEQ ID NO: 1, SEQ ID NO: 2, SEQ ID NO: 8 or SEQ ID NO: 9.
55. The method of points 53 or 54, wherein the polypeptide comprises an additional methionine at the N terminus; and/or a His tag at the C terminus or at the N-terminus.
56. The method of any of points 53-55, wherein the polypeptide comprises or consists of a sequence selected from the group consisting of SEQ ID NOs: 3, 4, 5, 6, 7, 10, and 11.
57. The method of any of points 56, wherein the polypeptide comprises or consists of SEQ ID NO: 7.
58. The method of any of points 41-57, wherein (ii)(a) further comprises treating the sample with a further enzyme to inactivate an antimicrobial substance in the sample.
59. The method of point 58, wherein the further enzyme is a beta-lactamase.
60. The method of point 59, wherein the beta-lactamase is a carbapenemase.
61. The method of point 60, wherein the carbapenemase is a class B carbapenemase.
62. The method of point 61, wherein the class B carbapenemase comprises a New Delhi metallo-beta-lactamase 1 (NDM-1) polypeptide.
63. The method of point 62, wherein the New Delhi metallo-beta-lactamase 1 (NDM-1) polypeptide comprises a further polypeptide having at least 95% sequence identity to SEQ ID NO: 27 or 28.
64. The method of point 63, wherein the New Delhi metallo-beta-lactamase 1 (NDM-1) polypeptide comprises a further polypeptide having at least 95% sequence identity to SEQ ID NO: 28.
65. A surrogate efficacy test method for testing the efficacy of a vaccine, comprising:
   (i) obtaining a surrogate endpoint, comprising:
      (a) obtaining at least one sample associated with at least one of:
         (i) a first group of infants not afflicted by a disease or an infection after being exposed to a microbe, or
         (ii) a second group of infants afflicted by a disease or an infection after being exposed to a microbe, and
      (b) obtaining a measurement of immunological activity of the at least one sample, comprising:
         (i) removing, inactivating, and/or cleaving human IgM in the at least one sample,
         (ii) obtaining a measurement of immunological activity of the at least one sample against a microbe, and
      (c) determining the surrogate endpoint based on a plurality of measurements, and
   (ii) vaccinating at least one human using a vaccine against the microbe, and
   (iii) obtaining a measurement of immunological activity of a sample from the at least one vaccinated human, comprising:
      (a) removing, inactivating, and/or cleaving human IgM in the sample,
      (b) obtaining a measurement of immunological activity of the sample from the at least one vaccinated human against a microbe, and
   (iv) comparing the measurement obtained with the surrogate endpoint.
66. The method of point 65, wherein the microbe is a GBS bacterium.
67. The method of point 66, wherein the GBS bacterium expresses the surface proteins Rib or Alp2.
68. The method of point 67, wherein the GBS bacterium expresses the surface protein Rib.
69. The method of point 68, wherein the GBS bacterium expresses the surface protein Alp2.
70. The method of point 65, wherein the GBS bacterium belongs to the Rib/CPSIII or Alp2/CPSV GBS serotype.
71. The method of point 70, wherein the GBS bacterium belongs to the Rib/CPSIII GBS serotype.
72. The method of point 70, wherein the GBS bacterium belongs to the Alp2/CPSV GBS serotype.
73. The method of any of the points 65-72, wherein the measurement of immunological activity is obtained using an Opsonophagocytic killing Assay (OPkA).
74. The method of any of points 65-73, wherein (i)(b)(i) or (iii)(b) are performed by treating the sample with an enzyme to cleave human IgM in the sample.
75. The method of point 74, wherein (i)(b)(i) is performed by treating the sample with an enzyme to cleave human IgM in the sample.
76. The method of point 74, wherein (iii)(b) are performed by treating the sample with an enzyme to cleave human IgM in the sample.
77. The method of any of points 74-76, wherein the enzyme is from *Lachnoanaerobaculum sp. ICM7, Mediterraneibacter massiliensis, Lachnospiraceae bacterium,* or *Oribacterium Sinus.*
78. The method of any of points 74-76, wherein the gene encoding the enzyme is selected from the group consisting of accession IDs: EJP19400.1, WP_162858205.1, RKW40033.1, and WP_007157024.1.
79. The method of any of points 74-76, wherein the enzyme comprises or consists of a polypeptide having at least 80% sequence identity to SEQ ID NO: 1, SEQ ID NO: 2, SEQ ID NO: 8 or SEQ ID NO: 9.
80. The method of any of points 74-76, wherein the enzyme comprises or consists of a polypeptide having at least 90% sequence identity to SEQ ID NO: 1, SEQ ID NO: 2, SEQ ID NO: 8 or SEQ ID NO: 9.
81. The method of points 79 or 80, wherein the polypeptide comprises an additional methionine at the N terminus; and/or a His tag at the C terminus or at the N-terminus.
82. The method of any of points 79-81, wherein the polypeptide comprises or consists of a sequence selected from the group consisting of SEQ ID NOs: 3, 4, 5, 6, 7, 10, and 11.
83. The method of any of point 82, wherein the polypeptide comprises or consists of SEQ ID NO: 7.
84. The method of any of points 77-83, wherein (i)(b)(i) or (iii)(b) further comprise treating the sample with a further enzyme to inactivate an antimicrobial substance in the sample.
85. The method of point 84, wherein (i)(b)(i) further comprises treating the sample with a further enzyme to inactivate an antimicrobial substance in the sample.
86. The method of point 84, wherein (iii)(b) further comprises treating the sample with a further enzyme to inactivate an antimicrobial substance in the sample.
87. The method of any of points 84-86, wherein the further enzyme is a beta-lactamase.
88. The method of point 87, wherein the beta-lactamase is a carbapenemase.
89. The method of point 88, wherein the carbapenemase is a class B carbapenemase.
90. The method of point 89, wherein the class B carbapenemase comprises a New Delhi metallo-beta-lactamase 1 (NDM-1) polypeptide.
91. The method of point 90, wherein the New Delhi metallo-beta-lactamase 1 (NDM-1) polypeptide comprises a further polypeptide having at least 95% sequence identity to SEQ ID NO: 27 or 28.
92. The method of point 91, wherein the New Delhi metallo-beta-lactamase 1 (NDM-1) polypeptide comprises a further polypeptide having at least 95% sequence identity to SEQ ID NO: 28.
93. A kit comprising:
   (i) an enzyme to cleave human IgM in a sample, and
   (ii) a further enzyme to inactivate an antimicrobial substance in the sample.
94. The kit of point 93, wherein the enzyme is from *Lachnoanaerobaculum sp. ICM7, Mediterraneibacter massiliensis, Lachnospiraceae bacterium,* or *Oribacterium Sinus.*
95. The kit of point 93, wherein the gene encoding the enzyme is selected from the group consisting of accession IDs: EJP19400.1, WP_162858205.1, RKW40033.1, and WP_007157024.1.
96. The kit of point 93, wherein the enzyme comprises or consists of a polypeptide having at least 80% sequence identity to SEQ ID NO: 1, SEQ ID NO: 2, SEQ ID NO: 8 or SEQ ID NO: 9.
97. The kit of point 93, wherein the enzyme comprises or consists of a polypeptide having at least 90% sequence identity to SEQ ID NO: 1, SEQ ID NO: 2, SEQ ID NO: 8 or SEQ ID NO: 9.
98. The kit of points 96 or 97, wherein the polypeptide comprises an additional methionine at the N terminus; and/or a His tag at the C terminus or at the N-terminus.
99. The kit of any of points 96-98, wherein the polypeptide comprises or consists of a sequence selected from the group consisting of SEQ ID NOs: 3, 4, 5, 6, 7, 10, and 11.
100. The kit of any of points 96-98, wherein the polypeptide comprises or consists of SEQ ID NO: 7.
101. The kit of any of points 93-100, wherein the further enzyme is a beta-lactamase.
102. The kit of point 101, wherein the beta-lactamase is a carbapenemase.
103. The kit of point 102, wherein the carbapenemase is a class B carbapenemase.
104. The kit of point 103, wherein the class B carbapenemase comprises a New Delhi metallo-beta-lactamase 1 (NDM-1) polypeptide.
105. The kit of point 104, wherein the New Delhi metallo-beta-lactamase 1 (NDM-1) polypeptide comprises a further polypeptide having at least 95% sequence identity to SEQ ID NO: 27 or 28.
106. The kit of point 105, wherein the New Delhi metallo-beta-lactamase 1 (NDM-1) polypeptide comprises a further polypeptide having at least 95% sequence identity to SEQ ID NO: 28.

## Claims

1. A method of treating a sample, wherein the sample comprises blood, serum, and/or plasma from a human infant or from a human umbilical cord, wherein the method comprises:
i. removing, inactivating, and/or cleaving human IgM in the sample.

2. The method according to claim 1, wherein step (i) is performed by treating the sample with an enzyme to cleave human IgM in the sample.

3. The method according to claim 2, wherein the enzyme is from any of *Lachnoanaerobaculum sp. ICM7, Mediterraneibacter massiliensis, Lachnospiraceae bacterium,* and *Oribacterium Sinus,* preferably wherein the gene encoding the enzyme is selected from the accession IDs: EJP19400.1, WP_162858205.1, RKW40033.1, and WP_007157024.1.

4. The method according to claim 2, wherein the enzyme comprises or consists of a polypeptide having at least 80%, preferably at least 90%, sequence identity to SEQ ID NO: 1 or SEQ ID NO: 8, or alternatively at least 80%, preferably at least 90%, sequence identity to SEQ ID NO: 2 or SEQ ID NO: 9.

5. The method according to claim 4, wherein the polypeptide has an additional methionine at the N terminus; and/or a His tag at the C terminus or at the N-terminus.

6. The method according to any of the claims 4-5, wherein the polypeptide comprises or consists of a sequence selected from the group consisting of SEQ ID NOs: 3, 4, 5, 6, 7, 10, and 11, wherein the polypeptide preferably comprises or consists of SEQ ID NO: 7.

7. The method according to any of the preceding claims, further comprising the step of:
ii. treating the sample with a further enzyme to inactivate an antimicrobial substance in the sample, wherein the further enzyme preferably is a beta-lactamase, preferably a carbapenemase, more preferably a class B carbapenemase.

8. The method according to claim 7, wherein the further enzyme is a New Delhi metallo-beta-lactamase 1 (NDM-1) polypeptide comprising or consisting of a further polypeptide having at least 95% sequence identity to SEQ ID NO: 27 or 28, more preferably having at least 95% sequence identity to SEQ ID NO: 28.

9. A method of obtaining a measurement of immunological activity of a sample, comprising the steps of:
i. performing the method according to any preceding claim, and
ii. obtaining a measurement of immunological activity of the sample against a microbe.

10. The method according to claim 9, wherein the microbe is GBS bacteria, preferably wherein the GBS bacteria belong to a GBS strain expressing at least one of the GBS surface proteins Rib and Alp2, more preferably wherein the GBS bacteria belongs to Rib/CPSIII or Alp2/CPSV

11. The method according to any of the claims 8-9, wherein the measurement is obtained using an Opsonophagocytic killing Assay (OPkA).

12. A method of obtaining a surrogate endpoint, comprising the steps of:
i. performing the method according to claim 9 to obtain a measurement of immunological activity against a microbe for at least one sample associated with at least one of:
a. a first group of infants not afflicted by disease and/or infection after having been exposed to a microbe and
b. a second group of infants afflicted by disease and/or infection after being exposed to a microbe, and
ii. determining at least one surrogate endpoint based on the measurement.

13. A surrogate efficacy test method for testing efficacy of a vaccine comprising the steps of:
i. providing at least one surrogate endpoint by performing the method according to claim 12,
ii. vaccinating at least one human using a vaccine against the microbe,
iii. performing the method according to any of the claims 9-11 on a sample from the at least one human or from the offspring of the at least one human, and
iv. comparing the measurement obtained with the at least one surrogate endpoint.

14. Use of an enzyme as defined in any of the claims 3-6 to inactivate and/or cleave human IgM in a sample comprising blood, serum, and/or plasma from a human infant or from a human umbilical cord.

15. A kit comprising:
- an enzyme as defined in any of the claims 3-6, and
- a further enzyme as defined in any of the claims 7-8.
